(19) Europäisches Patentamt
European Patent Office
Office européen des brevets

(11) **EP 3 163 481 B1**

(12) **EUROPEAN PATENT SPECIFICATION**

(45) Date of publication and mention of the grant of the patent:
**01.05.2019 Bulletin 2019/18**

(21) Application number: **15812344.8**

(22) Date of filing: **24.06.2015**

(51) Int Cl.:
*G16B 20/10* (2019.01)   *G16B 30/10* (2019.01)

(86) International application number:
**PCT/JP2015/068277**

(87) International publication number:
**WO 2015/199162 (30.12.2015 Gazette 2015/52)**

(54) **DEVICE, METHOD AND PROGRAM FOR PREDICTING MUTANT CAPABLE OF THERMALLY STABILIZING MEMBRANE PROTEIN**

VORRICHTUNG, VERFAHREN UND PROGRAMM ZUR VORHERSAGE VON MUTANTEN ZUR WÄRMESTABILISIERUNG EINES MEMBRANPROTEINS

DISPOSITIF, PROCÉDÉ ET PROGRAMME DE PRÉDICTION DE MUTANT CAPABLE DE STABILISER THERMIQUEMENT UNE PROTÉINE DE MEMBRANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2014 JP 2014130560**

(43) Date of publication of application:
**03.05.2017 Bulletin 2017/18**

(73) Proprietor: **Japan Science and Technology Agency**
**Kawaguchi-shi**
**Saitama 332-0012 (JP)**

(72) Inventors:
• **MURATA, Takeshi**
**Chiba-shi**
**Chiba 263-8522 (JP)**
• **KINOSHITA, Masahiro**
**Uji-shi**
**Kyoto 611-0011 (JP)**
• **YASUDA, Satoshi**
**Uji-shi**
**Kyoto 611-0011 (JP)**
• **TAKAMUKU, Yuuki**
**Chiba-shi**
**Chiba 263-8522 (JP)**
• **MIZUTANI, Kenji**
**Chiba-shi**
**Chiba 263-8522 (JP)**
• **SUZUKI, Nanao**
**Chiba-shi**
**Chiba 263-8522 (JP)**
• **KAJIWARA, Yuta**
**Uji-shi**
**Kyoto 611-0011 (JP)**

(74) Representative: **Cabinet Plasseraud**
**66, rue de la Chaussée d'Antin**
**75440 Paris Cedex 09 (FR)**

(56) References cited:
WO-A1-2012/040833    JP-A- 2010 134 514
JP-A- 2012 504 801    US-A1- 2013 013 279

• **PARK H ET AL: "Prediction of the mutation-induced change in thermodynamic stabilities of membrane proteins from free energy simulations", BIOPHYSICAL CHEMISTRY, ELSEVIER, AMSTERDAM, NL, vol. 114, no. 2-3, 22 April 2005 (2005-04-22), pages 191-197, XP027605128, ISSN: 0301-4622 [retrieved on 2005-04-22]**
• **Anna Johansson: "Solvation properties of proteins in membranes", Doctoral Thesis, 22 May 2009 (2009-05-22), XP055435654, Retrieved from the Internet: URL:https://www.diva-portal.org/smash/get/diva2:214204/FULLTEXT01.pdf [retrieved on 2017-12-15]**

**Description**

Field

The present disclosure relates to a thermostabilized mutant-predicting apparatus for membrane proteins, a thermostabilized mutant-predicting method, and a computer program product.

Background

Membrane proteins occupy 30% of all of proteins to be encoded by genomes and play an important role in cell functions such as signal transduction, substance transportation, bio-energy production and conversion. At the same time, since about 60% of commercially available drugs affect membrane proteins, a membrane protein is an important target in medicinal drug discovery. In particular, G protein-coupled receptor (GPCR), which is a receptor for hormones, neurotransmitters and the like, forms about 800 kinds of families, and among them about 280 kinds are estimated as targets for drug discovery.

Recently, for designing and improving effective drugs with less side effect, Structure-Based Drug Design(SBDD) based on the three-dimensional structure of protein as a drug target has been considered effective. However, GPCR has problems like (1) the thermal stability is inferior and thus mass production is difficult; and (2) the hydrophilic surface for crystallization is small and thus crystallization is difficult. As a result, any detailed structure of human GPCR was not obtained until the year 2007.

In 2007, as described in Non-patent literature 1, a group of B.Kobilka and R.Stevens et al. succeeded in X-ray crystallography by fusing T4 lysozyme (T4L) with an intracellular third loop of human adrenergic receptor, thereby thermally stabilizing the receptor and at the same time extending the hydrophilic surface, and crystalizing by a method that is called a lipidic cubic phase method. That is, the problem of the GPCR (2), namely difficulty in crystallization, can be overcome by use of T4L fusion and antibody. However, the problem (1), namely inferior thermal stability to hinder mass production, cannot be solved sufficiently, and even now 90% or more of the GPCR cannot be produced at a large scale.

In Patent literature 1 or the like, StaR (registered trademark) technique is disclosed. According to the StaR (registered trademark) technique, for enhancing the thermal stability, the respective amino acids of GPCR are substituted exhaustively by alanine, any mutation sites that will improve the thermal stability are examined experimentally, these mutation sites are combined to remarkably improve the thermal stability and the crystal structure is analyzed, and the thermal stability for the other type of GPCR is also improved by using the similarity of the GPCR structure.

Citation List

Patent Literature

Patent Literature 1: JP-T-2011-505800

Non-Patent Literature

Non-Patent Literature 1: Vadim Cherezov, Daniel M. Rosenbaum, Michael A. Hanson, Soren G. F. Rasmussen, Foon Sun Thian, Tong Sun Kobilka, Hee-Jung Choi, Peter Kuhn, William I. Weis, Brian K. Kobilka, Raymond C. Stevens, "High-Resolution Crystal Structure of an Engineered Human β2-Adrenergic G Protein-Coupled Receptor", Science 2007, Vol.318 no.5854 pp. 1258-1265

Summary

Technical Problem

However, since the StaR (registered trademark) technique requires experimental exhaustive analysis, the effect of substitution of amino acids other than alanine has not been known. Another problem is that this technique requires considerable time and effort to newly perform a similar analysis for any other membrane proteins such as GPCR.

It is an object of the present disclosure to at least partially solve the aforementioned problems in the conventional technology, by providing a thermostabilized mutant-predicting apparatus capable of predicting with computer any amino acid mutant for thermal stabilization in a membrane protein, a thermostabilized mutant-predicting method, and a computer program product.

Advantageous Effects of Disclosure

A predicting apparatus according to one aspect of the present disclosure is a predicting apparatus that predicts a change in solvation entropy of a membrane protein by amino acid substitution, and the predicting apparatus includes a storage unit and a control unit. The storage unit ios configured to store an amino acid sequence of the membrane protein. The control unit includes a mutation-introducing unit configured to introduce an amino acid mutation into the amino acid sequence of the membrane protein to create an amino acid sequence of an amino acid mutant, a calculating unit configured to calculate for each amino acid mutant a solvation entropy change occurring during formation, within a transmembrane segment, of a tertiary structure from a primary structure or from units in a secondary structure. The calculating unit is also configured to calculate solvation entropy (S) by the following formula:

$$S/k_B \ = \ C_1 V + C_2 A + C_3 X + C_4 Y,$$

where V is the excluded volume, A is the accessible surface area, X is the integrated value of mean curvature of accessible surface and Y is the integrated value of Gaussian curvature of accessible surface,
wherein $C_1$, $C_2$, $C_3$ and $C_4$ are determined by a least square method applied to the following formula:

$$S'/k_B = C_1 (4\pi R^3/3) + C_2 (4\pi R^2) + C_3 (4\pi R) + C_4 (4\pi),$$

where $R=(d_U+d_S)/2$, where $d_S$ is a solvent molecule diameter, and $d_U$ is a spherical (rigid sphere) solute diameter and where S' is the solvation entropy of isolated rigid-sphere solute having various diameters which is calculated by using the integral equation theory.

The calculating unit may be further configured to calculate for the membrane protein a solvation entropy change occurring during formation, within a transmembrane segment, of a tertiary structure from a primary structure or from units of a secondary structure.

The predicting apparatus according to still another aspect of the present invention is the predicting apparatus, wherein the storage unit further configured to store structural data of the membrane protein, and the calculating unit is configured to perform structural optimization based on the amino acid sequence and the structural data.

The predicting apparatus according to still another aspect of the present invention is the predicting apparatus, wherein the calculating unit is configured to perform the structural optimization while relaxing a constraint stepwise, by first fixing heavy atoms of the membrane protein and minimizing, then fixing Cα carbon and Cβ carbon and minimizing, and finally minimizing without fixation.

The predicting apparatus according to still another aspect of the present invention is the predicting apparatus, wherein the calculating unit is configured to calculate, as the solvation entropy change, a difference between the solvation entropy of the tertiary structure obtained by first structural optimization then extraction of the transmembrane segment, and the solvation entropy of the secondary structure obtained by pulling apart the tertiary structure.

The predicting apparatus according to still another aspect of the present invention is the predicting apparatus, wherein the calculating unit is configured to calculate, as the solvation entropy change, a difference between solvation entropy of the tertiary structure obtained by first extraction of the transmembrane segment then structure optimization, and solvation entropy of the secondary structure obtained by first pulling apart the tertiary structure then structural optimization.

The predicting apparatus according to still another aspect of the present invention is the predicting apparatus, wherein the calculating unit is configured to calculate, as the solvation entropy change, a difference between solvation entropy of the tertiary structure obtained by first structural optimization then extraction of the transmembrane segment, and solvation entropy of the secondary structure obtained by first extraction of the transmembrane segment then pulling apart transmembrane segment then structural optimization.

The predicting apparatus according to still another aspect of the present invention is the predicting apparatus, wherein the calculating unit is configured to calculate, as the solvation entropy change, a difference between solvation entropy of the tertiary structure obtained by first structural optimization then extraction of the transmembrane segment, and solvation entropy of the primary structure obtained by first extraction of the transmembrane segment then stretching the transmembrane segment then structural optimization.

The predicting apparatus according to still another aspect of the present invention is the predicting apparatus, wherein the calculating unit is configured to calculate, as the solvation entropy change, a difference between solvation entropy of the tertiary structure obtained by extraction of the tranmembrane segment then structural optimization, and solvation entropy of the primary structure obtained
by extraction of the transmembrane segment then stretching the transmembrane segment then structural optimization.

The predicting apparatus according to still another aspect of the present invention is the prediction apparatus, wherein the control unit further comprises a candidate-extracting unit configured to extract a candidate of the amino acid mutant to be thermostabilized, based on a value of the difference between the solvation entropy change in the membrane protein and the solvation entropy changes in the amino acid mutant, an amino acid mutant being extracted as a candidate of the amino acid mutant to be thermostabilized if the value is a negative value. The candidate-extracting unit may be further configured to extract a candidate of the amino acid mutant to be thermostabilized, based on a value of the difference between the solvation entropy change in the membrane protein and the solvation entropy change in the amino acid mutant, the amino acid mutant being extracted as a candidate of the amino acid mutant to be thermostabilized if the value is equal to or less than a certain value.

The calculating unit may be further configured to calculate for the membrane protein and each amino acid mutant an energy change in formation of the tertiary structure from the primary structure or formation of the tertiary structure from secondary-structure units within the transmembrane segment involving the structural optimization based on the amino acid sequence; and the candidate-extracting unit may be further configured to extract the candidate of the amino acid mutant to be thermostabilized, based on a change amount as a sum of a difference between the energy change in the membrane protein and the energy change in the amino acid mutant, and a value obtained by multiplying an absolute temperature to the difference between the solvation entropy change in the membrane protein and the solvation entropy change in the amino acid mutant.

The present invention also relates to a predicting method for predicting a change in solvation entropy of a membrane protein by amino acid substitution , which is executed in a computer including a storage unit for storing an amino acid sequence of the membrane protein and a control unit. The method includes a mutation-introducing step of introducing an amino acid mutation into the amino acid sequence of the membrane protein to create an amino acid sequence of the amino acid mutant, a calculating step of calculating, for each amino acid mutant, a solvation entropy change occurring during formation, within a transmembrane segment, of a tertiary structure from a primary structure or from units of secondary structure , wherein solvation entropy (S) is calculated by the following formula:

$$S/k_B \ = \ C_1V + C_2A + C_3X + C_4Y,$$

where V, A, X and Y are as defined above, and $C_1$, $C_2$, $C_3$ and $C_4$ determined as defined above.

The present invention also relates to a computer program product having a non-transitory tangible computer readable medium including programmed instructions for causing, when executed by a computer including a storage unit for storing an amino acid sequence of the membrane protein and a control unit, to perform the predicting method as defined above.

Further, the present disclosure relates to a recording medium on which the program is recorded.

Advantageous Effects of Disclosure

The present disclosure provides an effect of enabling prediction in silico of an amino acid mutation for thermal stabilization in a membrane protein, by storing an amino acid sequence of the membrane protein, introducing an amino acid mutation into the amino acid sequence of the membrane protein thereby creating an amino acid sequence of the amino acid mutant, then, for the membrane protein and each amino acid mutant, calculating a solvation entropy change in formation of a tertiary structure from a primary structure or formation of the tertiary structure from secondary-structure units within the transmembrane segment involving structural optimization based on the amino acid sequence, and extracting a candidate of an amino acid mutant to be thermostabilized based on a difference between the solvation entropy change in the membrane protein and the solvation entropy change in the amino acid mutant.

Further, the present disclosure provides an effect of enabling higher speed calculation of the solvation entropy by morphometrically simplifying the solute, which is achieved by calculating the solvation entropy change by using an integrated methodology of an integral equation theory and a morphometric representation based on four geometric indices of an excluded volume, an accessible surface area, and integrated mean and Gaussian curvatures of accessible surface.

Further, the present disclosure provides an effect of enabling an accurate optimization of the structure by utilizing known structural data since in the present disclosure the structural data of the membrane protein are stored and the structural optimization is performed based on the amino acid sequence and the structural data.

Further, the present disclosure provides an effect of obtaining a structure predicted more precisely by performing structural optimization while relaxing a constraint stepwise, by first fixing heavy atoms of a membrane protein and minimizing, next fixing C$\alpha$ carbon and C$\beta$ carbon and minimizing, and finally minimizing without fixation.

Further, the present disclosure can provide an effect of obtaining a comparatively high prediction hit rate (5/11 in an example), by calculating, as a solvation entropy change, a difference between solvation entropy of a tertiary structure subjected to structural optimization before extracting a transmembrane segment, and solvation entropy of a secondary structure from which the tertiary structure has been separated.

Further, the present disclosure can provide an effect of obtaining a high prediction hit rate (1/4 in an example), by calculating, as a solvation entropy change, a difference between solvation entropy of a tertiary structure subjected to structural optimization after extracting a transmembrane segment and solvation entropy of a secondary structure subjected to structural optimization after separating the extracted transmembrane segment.

Further, the present disclosure can provide an effect of obtaining a high prediction hit rate (3/11 in an example), by calculating, as a solvation entropy change, a difference between solvation entropy of a tertiary structure subjected to structural optimization before extracting a transmembrane segment and solvation entropy of a secondary structure subjected to structural optimization after extracting a transmembrane segment and separating the transmembrane segment.

Further, the present disclosure can provide an effect of obtaining a high prediction hit rate (5/11 in an example), by calculating, as a solvation entropy change, a difference between solvation entropy of a tertiary structure subjected to structural optimization before extracting a transmembrane segment and solvation entropy of a primary structure subjected to structural optimization after extracting a transmembrane segment and extending the transmembrane segment.

Further, the present disclosure can provide an effect of obtaining a high prediction hit rate (2/4 in an example), by calculating, as a solvation entropy change, a difference between solvation entropy of a tertiary structure subjected to structural optimization after extracting a transmembrane segment and solvation entropy of a primary structure subjected to structural optimization after extracting a transmembrane segment and separating and extending the transmembrane segment.

Brief Description of Drawings

FIG. 1 is a diagram of an example where an N atom is a donor and an O atom is an acceptor in a thermodynamic cycle ($T_0$=298K).
FIG. 2 is a diagram of an example where an N atom is a donor and an O atom is an acceptor in a thermodynamic cycle ($T_0$=298K).
FIG. 3 is a diagram schematically showing motions of rotation, diffusion, reversal and bending of phospholipid molecules in a lipid bilayer membrane.
FIG. 4 is a diagram schematically showing a sum R of radii of a hydrocarbon group and a spherical solute. With insertion of the spherical solute having 15 patterns of radii, 15 sets of (R,S) are obtained.
FIG. 5 is a diagram showing a two-stage model with respect to formation of a three-dimensional structure of a membrane protein.
FIG. 6 is a diagram of the native structure (NS) of GlycophorinA (GpA) formed of two structural units and decoy structures generated by a replica exchange Monte Carlo simulation.
FIG. 7 is a diagram of the native structure (NS) of GpA and 15000 decoy structures by plotting the root mean square deviation from the native structure on the x-axis and a nondimensionalized free energy difference on the y-axis.
FIG. 8 is a diagram of the native structure (NS) of GpA and 15000 decoy structures by plotting the root mean square deviation from the native structure on the x-axis and a nondimensionalized energy component difference on the y-axis.
FIG. 9 is a diagram of the native structure (NS) of GpA and 15000 decoy structures by plotting the root mean square deviation from the native structure on the x-axis and an entropy component (solvation entropy) difference on the y-axis.
FIG. 10 is a block diagram of an example of a thermostabilized mutant-predicting apparatus 100 to which the present embodiment is applied, conceptually showing only some of the units relating to the present embodiment.
FIG. 11 is a flow chart of an example of processing to be executed by the thermostabilized mutant-predicting apparatus 100.
FIG. 12 is a diagram schematically showing a method of calculating a solvation entropy change -$\Delta S_w$ in a membrane protein and a solvation entropy change -$\Delta S_m$ in an amino acid mutant in the thermostabilized mutant-predicting apparatus 100 of the present embodiment.
FIG. 13 is a diagram showing a course of separating a helix in Procedures 2 and 3 and then optimizing the structure.
FIG. 14 is a diagram of an example of a structure where the helix is extended to a primary structure in Procedures 4 and 5.
FIG. 15 is a flow diagram of a step common to Procedures 1 to 5 of the present embodiment.

EP 3 163 481 B1

FIG. 16 is a flow diagram of an example of processing of Procedure 1.
FIG. 17 is a flow diagram of an example of processing of Procedure 2.
FIG. 18 is a flow diagram of an example of processing of Procedure 3.
FIG. 19 is a flow diagram of an example of processing of Procedure 4.
FIG. 20 is a flow diagram of an example of processing of Procedure 5.
FIG. 21 is a diagram showing a calculation result (-$\Delta\Delta$S) by Procedures 1 to 5 for a mutant that is experimentally stabilized, where a threonine residue at position 88 has been substituted by glutamic acid.
FIG. 22 is a diagram showing a calculation result -$\Delta\Delta$S by Procedures 1 to 5 for a mutant that is experimentally stabilized (namely, its thermal denaturation temperature rises by 8°C), where a serine residue at position 91 has been substituted by arginine.
FIG. 23 is a diagram showing a calculation result -$\Delta\Delta$S by Procedures 1 to 5 for a mutant that is experimentally destabilized, where a cysteine residue at position 245 has been substituted by tryptophan.
FIG. 24 is a diagram showing a calculation result -$\Delta\Delta$S by Procedures 1 to 5 for a mutant that is experimentally destabilized, where an alanine residue at position 51 has been substituted by tryptophan.
FIG. 25 is a diagram showing a calculation result -$\Delta\Delta$S by Procedures 1 to 5 for a mutant that is experimentally destabilized, where a valine residue at position 239 has been substituted by arginine.
FIG. 26 is a table of a prediction result for the thermostabilized mutants by Procedures 1 to 5 with regard to five kinds of amino acid mutations.
FIG. 27 is a graph of a calculation result of -$\Delta\Delta$S in Procedure 1.
FIG. 28 is a graph of a calculation result of -$\Delta\Delta$S in Procedure 2.
FIG. 29 is a graph of a calculation result of -$\Delta\Delta$S in Procedure 3.
FIG. 30 is a graph of a calculation result of -$\Delta\Delta$S in Procedure 4.
FIG. 31 is a graph of a calculation result of -$\Delta\Delta$S in Procedure 5.
FIG. 32 is a table of a prediction result for a thermostabilized mutant for every amino acid mutation by Procedures 1 to 5.
FIG. 33 is a flow chart of an example of processing executed by the thermostabilized mutant-predicting apparatus.
FIG. 34 is a diagram showing energy lowering at the time of forming one intramolecular hydrogen bond.
FIG. 35 is a flow diagram of an example of processing of $\Lambda$.
FIG. 36 is a diagram of an example of prediction result.
FIG. 37 is a diagram of $\Delta\Delta$F values in Procedures 1 to 5 with respect to S91R.
FIG. 38 is a diagram of $\Delta\Delta$F values in Procedures 1 to 5 with respect to S91K.
FIG. 39 is a diagram of $\Delta\Delta$F values in Procedures 1 to 5 with respect to L85R.
FIG. 40 is a diagram of $\Delta\Delta$F values in Procedures 1 to 5 with respect to N280R.
FIG. 41 is a diagram of $\Delta\Delta$F values in Procedures 1 to 5 with respect to N181K.
FIG. 42 is a diagram of an example of prediction result.
FIG. 43 is a diagram of -$\Delta\Delta$S values in Procedures 1 to 5 with respect to S91R.
FIG. 44 is a diagram of -$\Delta\Delta$S values in Procedures 1 to 5 with respect to S91K.
FIG. 45 is a diagram of -$\Delta\Delta$S values in Procedures 1 to 5 with respect to L85R.
FIG. 46 is a diagram of -$\Delta\Delta$S values in Procedures 1 to 5 with respect to N280R.
FIG. 47 is a diagram of -$\Delta\Delta$S values in Procedures 1 to 5 with respect to N181K.

Description of Embodiments

Exemplary embodiments of a thermostabilized mutant-predicting apparatus, a thermostabilized mutant-predicting method, and a computer program product according to the present disclosure are described below in detail with reference to the accompanying drawings. The present embodiments are not intended to limit the present disclosure.

Summary of Embodiment

Hereinafter, for explaining the summary of embodiment of the present disclosure, first an integrated methodology of a morphometric representation and an integral equation theory developed by the inventors will be explained and then the summary, the constitution, the processing or the like of the present embodiments will be explained in detail.

Integrated Methodology

The present inventors focused on an entropy effect caused by translation of water molecules and succeeded in providing a picture of thermal denaturation of protein in an aqueous solution by an integrated methodology of morphometric approach and a statistical mechanics theory for liquids developed by the inventors.

The present disclosure aims to apply the methodology to focus on an entropy effect caused by the translation of $CH, CH_2$ and $CH_3$ groups (mass of these groups may be regarded as a solvent) constituting hydrophobic chains of phospholipid molecules, thereby theoretically predicting a change in solvation entropy of a membrane protein by amino acid substitution. Here, a free energy function F for the membrane protein is expressed by a formula below.

$$F = -TS + \Lambda$$

(T: absolute temperature, S: entropy component, $\Lambda$: energy component)

By dividing the above formula by $k_B T_0$ ($k_B$: Boltzmann's constant, $T_0 = 298K$) for nondimensionalization and setting $T = T_0$, a formula below is obtained.

$$F / (k_B T_0) = -S / k_B + \Lambda / (k_B T_0)$$

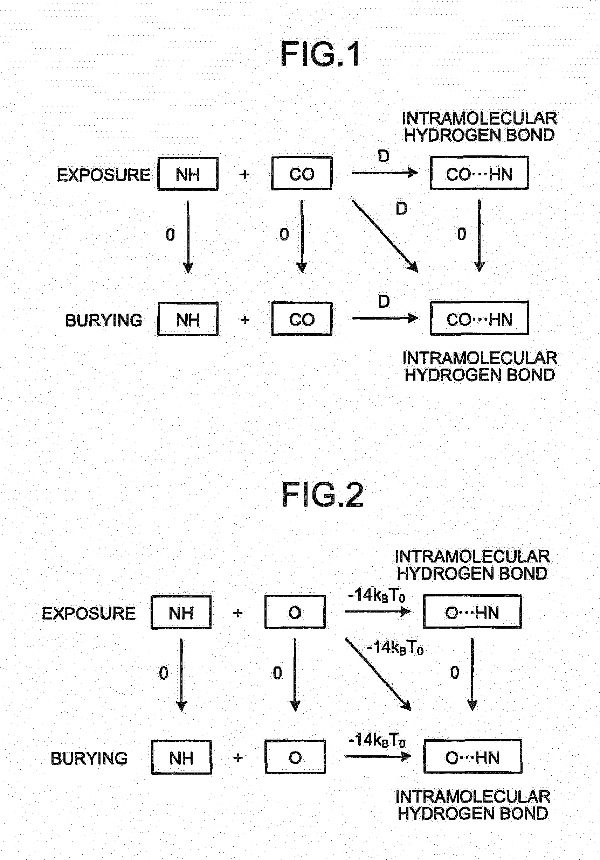

Here, FIG. 1 and FIG. 2 are diagrams of examples where an N atom is a donor and an O atom is an acceptor in a thermodynamic cycle ($T_0 = 298K$). As shown in FIG. 1, "energy lowering associated with formation of intramolecular hydrogen bond = the number of intramolecular hydrogen bonds *D" is calculated based on a completely-extended structure (having no intramolecular hydrogen bond).

Here, D is a value of energy lowering at the time of forming one intramolecular hydrogen bond. D may be a value of energy lowering (for example, $-14 k_B T_0$) obtained when formamide forms one hydrogen bond in a nonpolar solvent.

For example, as shown in FIG. 2, "energy lowering associated with formation of intramolecular hydrogen bond = the number of intramolecular hydrogen bonds *D ($-14 k_B T_0$)" may be calculated based on a completely-extended structure (having no intramolecular hydrogen bond). Here, $-14 k_B T_0$ is an energy lowering value obtained when the formamide forms one hydrogen bond in a nonpolar solvent.

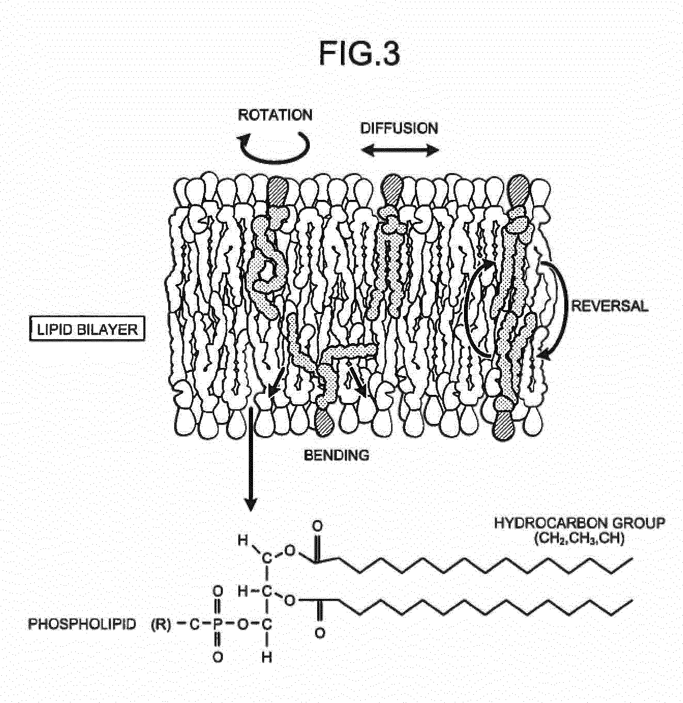

Then, every donor and acceptor between a main chain and a main chain, between a main chain and a side chain, and between a side chain and a side chain are examined, the number of intramolecular hydrogen bonds is counted to calculate "$\Lambda$ = energy lowering (negative) associated with formation of intramolecular hydrogen bond". It is assumed that acquisition of van der Waals attractive interaction within the protein molecule mutually cancel with loss of the van der Waals attractive interaction between protein and phospholipid. FIG. 3 is a diagram schematically showing the motions of rotation, diffusion, reversal and bending of phospholipid molecules in a lipid bilayer membrane (see LS-EDI Life Science Educational Digital Image Repository "Nature of lipid bilayer " by Tokyo University and Center for Structuring Life Science Network, URL:http://csls-db.c.u-tokyo.ac.jp/search/detail?image_repository_id=696).

As shown in FIG. 3, upon protein insertion into the membrane, loss of entropy occurs due to the restriction of thermal motions of the phospholipid molecules. The entropy is represented by a translational configurational entropy of a hydrocarbon group mass of phospholipid molecules. The degree of the loss is expressed as a function of three-dimensional structure, and a three-dimensional structure with smaller loss is more stable. Here, S is a translational configurational entropy loss (negative) of a hydrocarbon group mass associated with the insertion.

When the hydrocarbon group mass of the phospholipid molecules is regarded as a solvent, S is solvation entropy (entropy loss of a solvent that occurs when a solute with a fixed three-dimensional structure is inserted into the solvent: negative quantity).

In the present embodiment, a high-speed computation may be conducted in calculating the solvation entropy S by using the integrated methodology of the integral equation theory and the morphometric representation devised by the inventors.

According to the morphometric representation, solvation entropy of a three-dimensional structure of a solute can be calculated based on four geometric indices (an excluded volume V, an accessible surface area A, an integrated mean curvature X of accessible surface, and an integrated Gaussian curvature Y of accessible surface). That is, the expression of solvation entropy S is expressed by a linear combination below.

$$S / k_B = C_1 V + C_2 A + C_3 X + C_4 Y$$

Here, an excluded space is "a space which centers of solvent molecules cannot enter". The volume of the excluded space is the excluded volume V, and the surface area of the excluded space is the accessible surface area A. The excluded space also forms a conjugate of spheres of various radii, and contribution of a sphere having a radius r to X and Y is as follows.

"Contribution to X = Mean curvature 1/r multiplied by accessible surface area $\xi$ of the sphere; Contribution to Y = Gaussian

curvature $1/r^2$ multiplied by $\xi$".

According to the morphometrics, coefficients $C_1$, $C_2$, $C_3$ and $C_4$ of the four morphology indices do not rely on the geometric property of the solute, and thus, they can be processed in a simplified form (for example, sphere). Therefore, the form is regarded as a simplified sphere to calculate an entropy loss associated with insertion of spherical solutes having various diameters. In the present embodiment, the hydrocarbon group mass is modeled as a rigid sphere solvent and calculated by using an integral equation theory. According to morphometric representation with respect to the spherical solute, the following formulae are provided.

$$S/k_B = C_1(4\pi R^3/3) + C_2(4\pi R^2) + C_3(4\pi R) + C_4(4\pi),$$

$$R = (d_U + d_S)/2$$

Here, $d_S$ is a solvent molecule diameter, and $d_U$ is a spherical (rigid sphere) solute diameter. S of isolated rigid-sphere solute having various diameters (about 15 patterns within a range of $0 < d_U \leqq 30 d_S$) is calculated by using the integral equation theory, and $C_1$-$C_4$ are determined by a least squares method applied with the above formulae. Once the $C_1$-$C_4$ are determined, they are applied also to proteins having optional three-dimensional structures. Namely, S is obtained directly from the formulae by only calculating V, A, X, and Y, though $C_1$-$C_4$ rely considerably on the type of solvent and thermodynamic conditions (such as temperature and pressure).

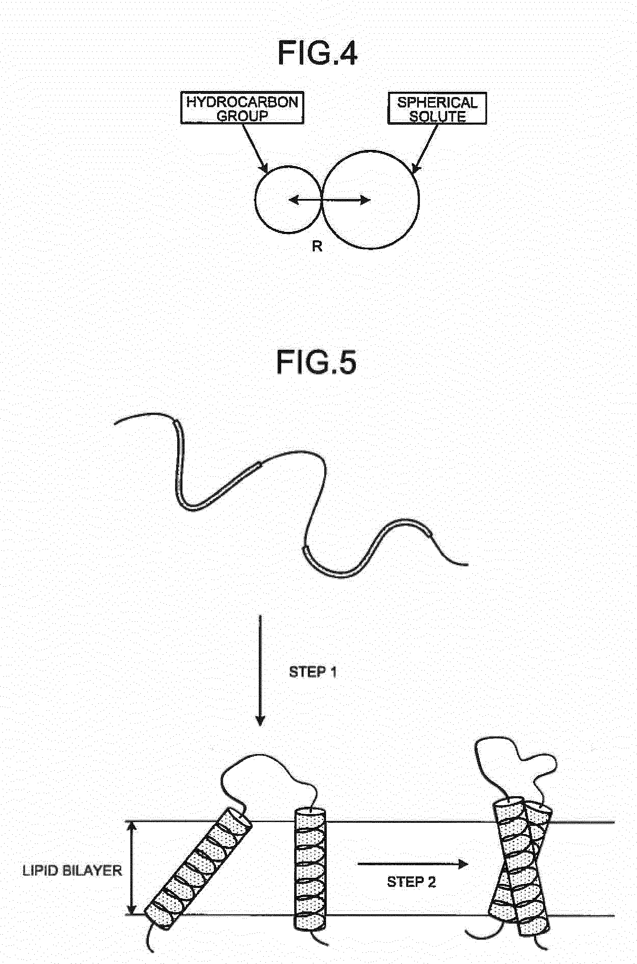

FIG. 4 is a diagram schematically showing a sum R of radii of the hydrocarbon group and the spherical solute. For example, 15 sets of (R,S) are obtained as a result of insertion of the spherical solute having 15 kinds of diameters.

When the solute is protein and a calculation using the above-described four geometric indices is performed for the diameters and x, y, z-coordinates of the constituent atoms (H, C, N, O, and S), S can be calculated at a high speed (within 1 second) even with a standard workstation per three-dimensional structure by the above-described integrated methodology of the statistical mechanics theory and the morphometric approach. When compared with a case of calculating with a three-dimensional integral equation theory in light of the complicated polyatomic structure, the calculation time is about 1/10,000, and the error is less than $\pm 5\%$. Even if the calculation of $\Lambda$ is included, the calculation itself of the free energy function F ends within 1 second.

As mentioned above, a high-speed computation with less processing load is available by applying the integrated methodology, which was developed regarding protein in an aqueous solution, to a membrane protein. Hereinafter, the integral equation theory will be explained.

The integral equation theory starts from the system partition function, and derives relational expressions established among various distribution functions (correlation functions) while defining the distribution functions. Regarding the equilibrium structures and physical properties, this process allows analyses of the same level as a computer simulation. Since this theory targets an indefinitely large system and takes the average of physical quantity with respect to an infinite number of microscopic states, the theory is free from problems such as "the system size may be too small; statistical error is inevitable".

In a case of a bulk solvent composed of a single component, relational expressions established among the distribution functions are solved numerically with input data of temperature, number density and interaction potential among solvent molecules, whereby microscopic structure and various thermodynamic quantities representing macroscopic properties of the solvent can be obtained (which can be extended to a solvent composed of multicomponents).

Solvation properties of a solute (microscopic structure of solvent in the vicinity of solute; the thermodynamic quantity of solvation) also can be analyzed. Here, the thermodynamic quantity of solvation indicates a change in thermodynamic quantity that occurs when a solute (three-dimensional structure is fixed) is inserted into the solvent. In a case of a simple solvent such as a rigid sphere system or Lennard-Jones fluids, a solute having an optional shape and polyatomic structure can be processed directly (three-dimensional integral equation theory). The integral equation theory gains an advantage over a computer simulation in calculation of thermodynamic quantity of the solvation. However, since a considerable amount of computational load is required for solving basic formulae, this is solved by integrating with the above-described morphometric indices in the present embodiment.

Summary of the present embodiment

Hereinafter, summary of the present embodiments will be described.

First in the present embodiment, an amino acid sequence of an amino acid mutant where respective amino acid residues of a membrane protein have been substituted by all of the amino acids other than Gly and Pro is created. For example, a membrane protein of a mutant can be obtained by introducing an amino acid mutation into a wild type membrane protein. The amino acid mutation may be an amino acid sequence formed by deleting, substituting or adding one or a plurality of amino acids from/for/to an original amino acid sequence.

Further in the present embodiment, an amino acid sequence of an amino acid mutant where the respective amino acid residues of a membrane protein have been substituted by all of amino acid including Gly and Pro may be created.

Next, in the present embodiment, for each of the amino acid mutants, the solvation entropy change $-\Delta S$ in formation of a tertiary structure from a primary structure or formation of the tertiary structure from secondary-structure units within the transmembrane segment involving structural optimization is calculated based on the amino acid sequence. FIG. 5 is a diagram showing a two-stage model with respect to formation of a three-dimensional structure of the membrane protein (see curr. opin. struct. biol. 2011, 21:460-466).

Stage 1 relates to a stage where a membrane protein forms secondary-structure units from its primary structure. More specifically, structural units of $\alpha$-helices are stabilized individually within the membrane and form as many intramolecular hydrogen bonds as possible (Step 1). In a lipid bilayer membrane, an $\alpha$ helix has the advantage over a $\beta$ sheet.

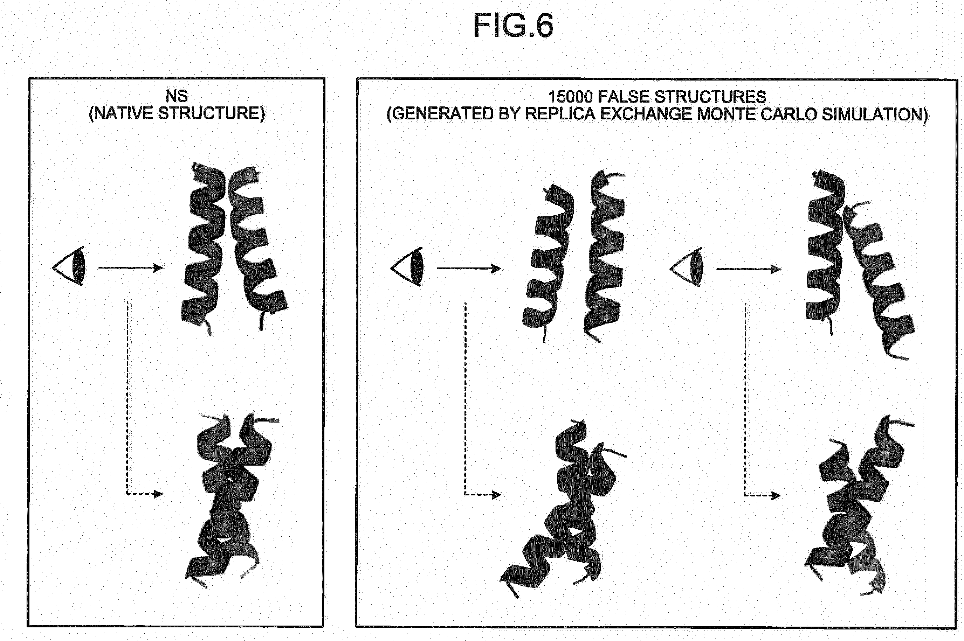

Stage 2 relates to a stage where the membrane protein forms its tertiary structure from secondary-structure units within the membrane. More specifically, side chains between structural units of $\alpha$-helices are closely packed (Step 2). In the present embodiment, a solvation entropy change up to formation of the tertiary structure from the primary structure through the Stages 1 and 2 may be calculated, or solvation entropy change up to formation of the tertiary structure from the secondary-structure units through the Stage 2 may be calculated. FIG. 6 is a diagram showing the native structure (NS) of GlycophorinA (GpA) composed of two structural units and decoy structures generated by a replica exchange Monte Carlo simulation. The structures below in the drawing show the same structures as shown above in the same drawing from the viewpoint of illustration. As shown in FIG. 6, the $\alpha$ helix structures themselves as secondary structures are the same but the positional relationship of the $\alpha$ helices in the tertiary structures are different from each other.

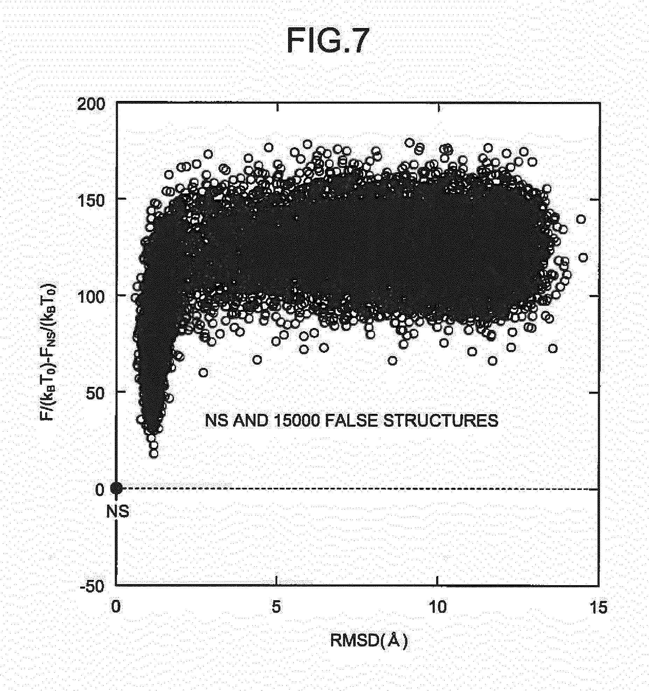

Then, a candidate of an amino acid mutant to be thermostabilized is extracted based on a difference $-\Delta\Delta S$ between the solvation entropy change $-\Delta S_w$ in a membrane protein wild type and solvation entropy change $-\Delta S_m$ in the amino acid mutant. FIG. 7 is a diagram plotting the root mean square deviation from the native structure on the x-axis and a nondimensionalized free energy difference on the y-axis for the native structure (NS) of GpA and 15000 decoy structures thereof.

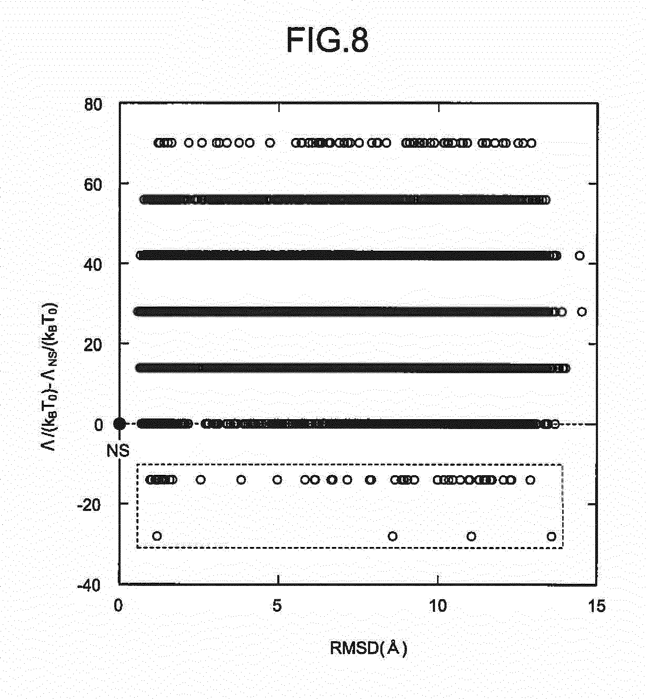

As shown in FIG. 7, the free energy F of the native structure takes the lowest value. FIG. 8 is a diagram plotting the root mean square deviation from the native structure on the x-axis and the nondimensionalized energy component difference on the y-axis for the native structure (NS) of GpA and 15000 decoy structures thereof.

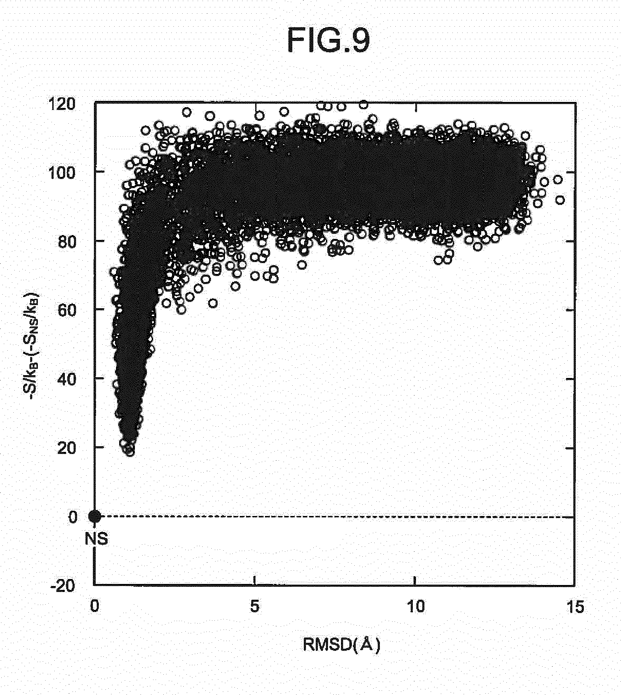

As shown in FIG. 8, numbers of decoy structures (indicated as dots inside the rectangle drawn with a broken line) advantageous over the native structure are present as false-positive since intramolecular hydrogen bond is not formed among the structural units. FIG. 9 is a diagram plotting the root mean square deviation from the native structure on the x-axis and the entropy component (solvation entropy) difference on the y-axis for the native structure (NS) of GpA and 15000 decoy structures thereof.

As shown in FIG. 9, the native structure can be extracted correctly without detecting false positive, based on the difference of the solvation entropy changes as an index in the present embodiment. The reason is that the side chains between the structural units are closely packed to maximize the entropy of the hydrocarbon group mass in the native structure.

The present embodiment is summarized above. Examples of the apparatus constitution and processing for executing the present embodiment of the present disclosure will be explained in detail below.

Constitution of Thermostabilized Mutant-predicting Apparatus

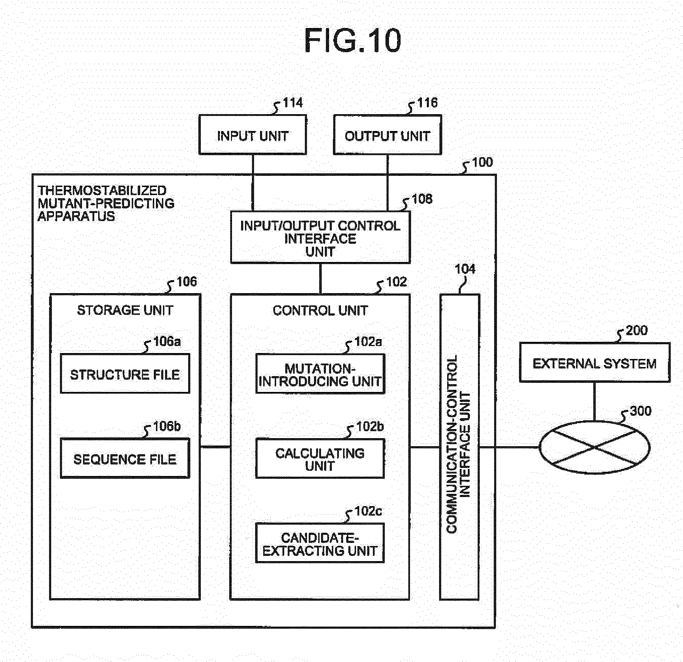

Next, the constitution of a thermostabilized mutant-predicting apparatus 100 in the present embodiment will be explained in detail with reference to FIG. 10. FIG. 10 is a block diagram of an example of the thermostabilized mutant-predicting apparatus 100 to which the present embodiment is applied, showing conceptually only the units relating to the present embodiment.

As shown in FIG. 10, the thermostabilized mutant-predicting apparatus 100 in the present embodiment schematically includes at least a control unit 102 and a storage unit 106, and in the present embodiment further includes an input/output control interface unit 108 and a communication-control interface unit 104. The control unit 102 is a Central Processing Unit (CPU) or the like that generally controls the entire thermostabilized mutant-predicting apparatus 100. Further, the communication-control interface unit 104 is an interface to be connected to a communication apparatus (not shown) such as a router to be connected to a communication circuit or the like, and the input/output control interface unit 108 is an interface to be connected to the input unit 114 and the output unit 116. And the storage unit 106 is a unit that stores various databases and tables. Each of these units of the thermostabilized mutant-predicting apparatus 100 is connected communicatively via optional communication paths. Furthermore, this thermostabilized mutant-predicting apparatus 100 is connected communicatively to a network 300 via a communication apparatus such as the router or a wire communication or wireless communication line such as an exclusive line.

The various databases and tables (structure file 106a and sequence file 106b or the like) to be stored in the storage unit 106 are storage units like a fixed disc device. For example, the storage unit 106 stores various programs, tables, files, databases and webpages to be used for various processing.

Among these components of the storage unit 106, the structure file 106a is a structural data storing unit that stores structural data of the membrane protein. The structure file 106a may store structural data or the like of a membrane protein that has been input via the input unit 114 and whose crystal structure has been analyzed. Structural data in the structure file 106a may include coordinates or the like of the respective atoms in the two-dimensional space and a three-dimensional space.

The sequence file 106b is a sequence data storing unit that stores sequence data of the membrane protein. The sequence file 106b may store sequence data or the like of the membrane protein that have been input via the input unit 114.

In FIG. 10, the input/output control interface unit 108 controls the input unit 114 and the output unit 116. For the output unit 116, a speaker as well as a monitor (including home television set) can be used (hereinafter, the output unit 116 may be described as a monitor). For the input unit 114, a keyboard, a mouse and a microphone can be used.

Further in FIG. 10, the control unit 102 has an internal memory for storing a control program such as an operating system (OS), a program that regulates various process steps or the like, and required data, and the control unit 102 performs information processing for executing various processes based on these programs. The control unit 102 includes a mutation-introducing unit 102a, a calculating unit 102b, and a candidate-extracting unit 102c functionally and conceptually.

As shown in FIG. 10, the mutation-introducing unit 102a is a mutation-introducing unit that introduces an amino acid mutation into each amino acid sequence of the membrane protein to create an amino acid sequence of an amino acid mutant (hereinafter, referred to simply as "mutant"). The mutation-introducing unit 102a may create an amino acid sequence as a mutant in which one or several amino acids are deleted from, substituted by or added to an original amino acid sequence.

The calculating unit 102b is a calculating unit that calculates solvation entropy changes $-\Delta S_w$ and $-\Delta S_m$ in formation of a tertiary structure from a primary structure or formation of the tertiary structure from secondary-structure units within the transmembrane segment involving structural optimization based on the amino acid sequence for the membrane protein wild type and each mutant. The calculating unit 102b may calculate the solvation entropy by using integrated methodology of integral equation theory and morphometric representation based on four geometric indices of an excluded volume V, an accessible surface area A, an integrated mean curvature X of accessible surface, and an integrated Gaussian curvature Y of accessible surface.

For the structural optimization, the calculating unit 102b may perform the structural optimization based on not only the amino acid sequence stored in the sequence file 106b but the structural data stored in the structure file 106a. Further, the calculating unit 102b may perform structural optimization while relaxing a constraint stepwise, by first fixing heavy atoms of the membrane protein and minimizing, and then fixing C$\alpha$ carbon and C$\beta$ carbon and minimizing, and finally minimizing without fixation. In addition to that, the calculating unit 102b may perform the structural optimization by using any other methods for structural optimization such as Modeller.

The calculating unit 102b may calculate the solvation entropy change by any of Procedure 1 to Procedure 5 below.

> Procedure 1: a difference between solvation entropy of a tertiary structure subjected to structural optimization before extracting a transmembrane segment, and solvation entropy of a secondary structure from which the tertiary structure has been separated;
> Procedure 2: a difference between solvation entropy of a tertiary structure subjected to structural optimization after extracting a transmembrane segment, and solvation entropy of a secondary structure subjected to structural optimization after separating the extracted transmembrane segment;
> Procedure 3: a difference between solvation entropy of a tertiary structure subjected to structural optimization before extracting a transmembrane segment, and solvation entropy of a secondary structure subjected to structural optimization after extracting a transmembrane segment and separating the transmembrane segment;
> Procedure 4: a difference between solvation entropy of a tertiary structure subjected to structural optimization before extracting a transmembrane segment, and solvation entropy of a primary structure subjected to structural optimization after extracting a transmembrane segment and extending the transmembrane segment; and
> Procedure 5: a difference between solvation entropy of a tertiary structure subjected to structural optimization after extracting a transmembrane segment, and solvation entropy of a primary structure subjected to structural optimization after extracting a transmembrane segment and separating and extending the transmembrane segment.

The candidate-extracting unit 102c is a candidate-extracting unit that extracts a candidate of a mutant to be thermostabilized based on a difference ($-\Delta\Delta S(=-\Delta S_w-(-\Delta S_m))$) between a solvation entropy change $-\Delta S_w$ in a membrane protein wild type and a solvation entropy change $-\Delta S_m$ in a mutant. For example, the candidate-extracting unit 102c may determine that the mutant is thermostabilized when $-\Delta\Delta S$ is a negative value and thermally destabilized when $-\Delta\Delta S$ is a positive value. In one example, the candidate-extracting unit 102c may extract a mutant whose $-\Delta\Delta S$ is equal to or less than a certain value as a candidate of a mutant to be thermostabilized. Hereinafter, each sign and the meaning are listed to correspond to each other.

S: solvation entropy (of a certain structure)
$-\Delta S$: a solvation entropy change (from a certain structure to another structure)
$-\Delta\Delta S$: a difference in a solvation entropy change (between protein before mutation and a mutant)

The above-described constitution is an example of the thermostabilized mutant-predicting apparatus 100 in the present embodiment. The thermostabilized mutant-predicting apparatus 100 may be connected to an external system 200 via the network 300. In this case, the communication-control interface unit 104 performs a communication control between the thermostabilized mutant-predicting apparatus 100 and the network 300 (or a communicating apparatus such as a router). Namely, the communication-control interface unit 104 has a function of communicating data with other terminals via a communication line. The network 300 has a function of interconnecting the thermostabilized mutant-predicting apparatus 100 and the external system 200, and for example, it is internet.

The external system 200 is interconnected to the thermostabilized mutant-predicting apparatus 100 via the network 300, and it has a function of providing external data base relating to various data such as structural data and sequence data, parameter and simulation result data, and a program for allowing a connected information processing apparatus to execute the thermostabilized mutant-predicting method.

The external system 200 may be constituted as a WEB server, an ASP server or the like. The hardware constitution of the external system 200 may be constituted with a commercially available information processing apparatus like work station and personal computers and their accessories. Further, the respective functions of the external system 200 are provided by the CPU, a disc device, a memory device, an input device, an output device, a communication-controlling apparatus and the like, and also a program or the like controlling thereof.

Constitutions in the present embodiment are as explained above.

Processing by Thermostabilized Mutant-predicting Apparatus 100

Next, an example of processing by the thus constituted thermostabilized mutant-predicting apparatus 100 in the present embodiment will be explained hereinafter in detail with reference to the drawings.

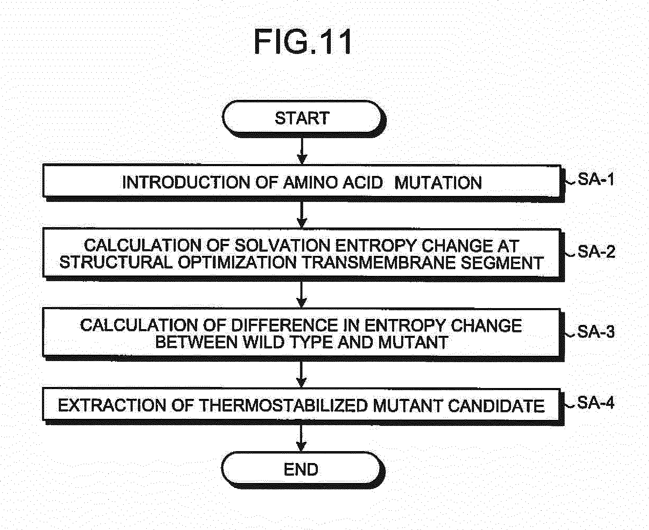

First, an example of processing executed by the thermostabilized mutant-predicting apparatus 100 is explained with reference to FIG. 11. FIG. 11 is a flow chart showing an example of processing executed by the thermostabilized mutant-predicting apparatus 100.

As shown in FIG. 11, first, the mutation-introducing unit 102a creates an amino acid sequence of a mutant Mt by introducing an amino acid mutation with respect to an amino acid sequence of a membrane protein stored in the sequence file 106b (Step SA-1). For example, the mutation-introducing unit 102a may create a mutant Mt into which any amino acid mutation such as one amino acid deletion, one amino acid substitution and one amino acid addition is introduced.

Then, the calculating unit 102b calculates the solvation entropy changes $-\Delta S_w$ and $-\Delta S_m$ in formation of the tertiary structure from the primary structure, or formation of the tertiary structure from secondary-structure units within the transmembrane segment involving structural optimization based on the amino acid sequence, for the membrane protein wild type Wt and the respective mutants Mt (Step SA-2). The calculating unit 102b may calculate the change in solvation entropy by any of Procedures 1 to 5 described below. Further, the calculating unit 102b may calculate the solvation entropy by using an integrated methodology of an integral equation theory and a morphometric representation based on four geometric indices of an excluded volume V, an integrated mean curvature X of accessible surface and an integrated Gaussian curvature Y of accessible surface. Regarding the structural optimization, the calculating unit 102b may perform the structural optimization based on not only the amino acid sequence stored in the sequence file 106b but the structural data stored in the structure file 106a. The calculating unit 102b may perform structural optimization while relaxing a constraint stepwise by first fixing the heavy atoms of the membrane protein and minimizing, then fixing C$\alpha$ carbon and C$\beta$ carbon and minimizing, and finally minimizing without fixation.

Then, the candidate-extracting unit 102c calculates a difference $-\Delta\Delta S(=-\Delta S_w-(-\Delta S_m))$ between the solvation entropy change $-\Delta S_w$ in the membrane protein wild type $-\Delta S_w$ and the solvation entropy change $-\Delta S_m$ in a mutant Mt (Step SA-3).

Then, the candidate-extracting unit 102c extracts a candidate of the mutant Mt to be thermostabilized, based on the calculated difference $-\Delta\Delta S$ (Step SA-4). For example, the candidate-extracting unit 102c may determine that the mutant is thermostabilized when the $-\Delta\Delta S$ is a negative value, and thermally destabilized when the $-\Delta\Delta S$ is a positive value. In one example, the candidate-extracting unit 102c may extract a mutant Mt having $-\Delta\Delta S$ equal to or lower than a predetermined threshold as a candidate of the mutant Mt to be thermostabilized.

An example of processing by the thermostabilized mutant-predicting apparatus 100 in the present embodiment is as explained above.

Five Kinds of Procedures

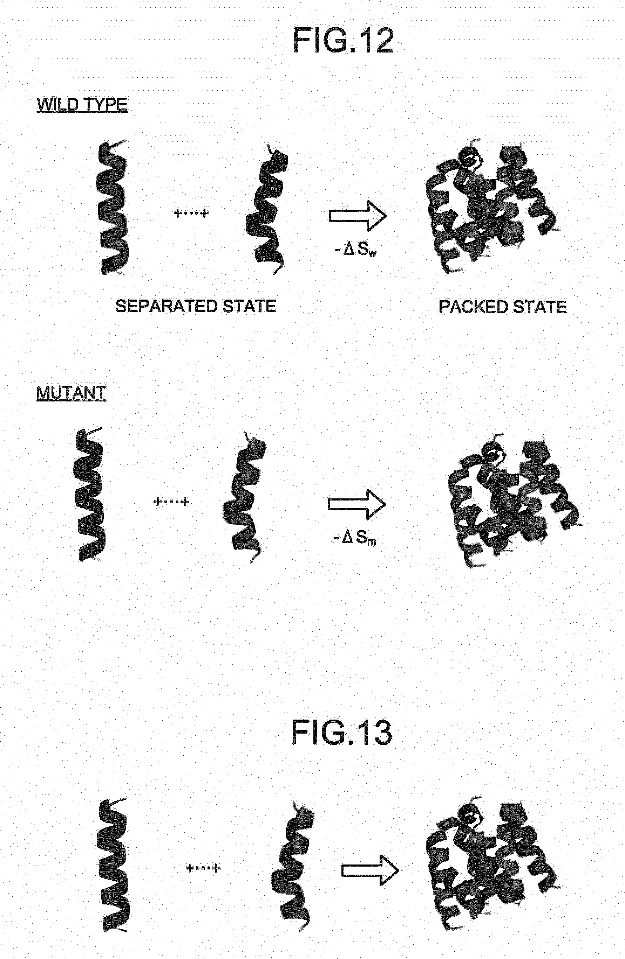

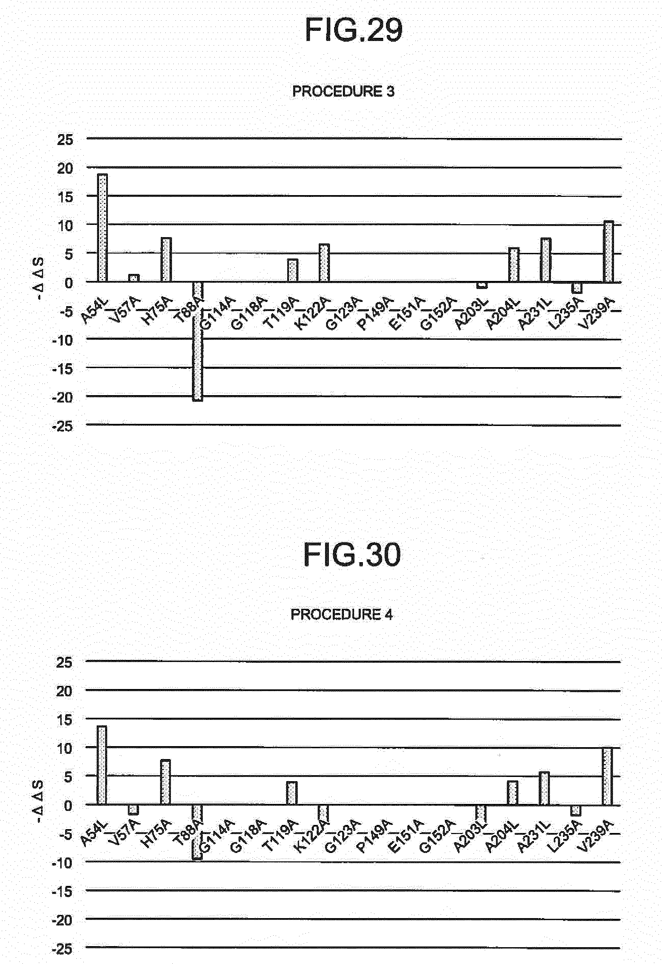

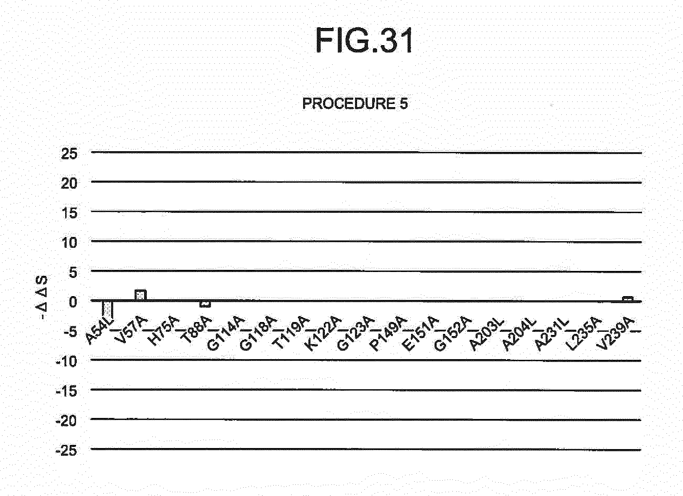

Hereinafter, processing of five kinds of Procedures 1 to 5 will be explained specifically in detail with reference to FIG. 12 to FIG. 32, based on the above-described processing. FIG. 12 is a diagram showing a method of calculating a solvation entropy change $-\Delta S_w$ in a membrane protein wild type Wt and a solvation entropy change $-\Delta S_m$ in a mutant Mt.

As a typical example, as shown in FIG. 12, for each of the membrane protein wild type Wt and the mutant Mt, a difference $-\Delta S$ between the solvation entropy of the state where the $\alpha$ helices are separated from each other and the solvation entropy of the state where the $\alpha$ helices are packed is calculated, as the solvation entropy change. And a difference $-\Delta\Delta S$ ($=-\Delta S_w-(-\Delta S_m)$) between the solvation entropy change $-\Delta S_w$ of the membrane protein wild type Wt and the solvation entropy change $-\Delta S_m$ of the mutant Mt is calculated.

Specifically, the calculating unit 102b may calculate the change in the solvation entropy by any of the following Procedures 1 to 5.

Procedure 1 is a method of calculating a difference $-\Delta S$ between solvation entropy S of a tertiary structure subjected to structural optimization before extracting a transmembrane segment, and solvation entropy S of a secondary structure from which the tertiary structure has been separated. In this manner, in Procedure 1, repacking closely the side chain of the separated helices is not taken into consideration.

Procedure 2 is a method of calculating a difference $-\Delta S$ between solvation entropy S of a tertiary structure subjected to structural optimization after extracting a transmembrane segment, and solvation entropy S of a secondary structure subjected to structural optimization after separating the extracted transmembrane segment. FIG. 13 is a diagram showing the helices being separated and then subjected to structural optimization in Procedures 2 and 3.

Procedure 3 is a method of calculating a difference $-\Delta S$ between solvation entropy S of a tertiary structure subjected to structural optimization before extracting a transmembrane segment, and solvation entropy S of a secondary structure subjected to structural optimization after extracting a transmembrane segment and separating the transmembrane segment. As shown in FIG. 13, in Procedures 2 and 3, each of the separated helices is subjected to structural optimization, and repacking closely the side chains is taken into consideration.

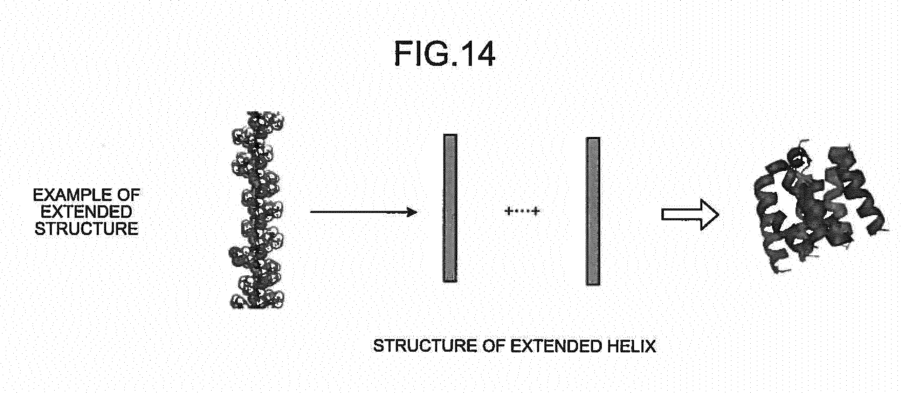

Procedure 4 is a method of calculating a difference $-\Delta S$ between solvation entropy S of a tertiary structure subjected to structural optimization before extracting a transmembrane segment, and solvation entropy S of a primary structure subjected to structural optimization after extracting a transmembrane segment and extending the transmembrane segment. FIG. 14 is a diagram showing an example of a structure where the helices are extended to a primary structure in Procedures 4 and 5.

Procedure 5 is a method of calculating a difference $-\Delta S$ between solvation entropy S of a tertiary structure subjected to structural optimization after extracting a transmembrane segment, and solvation entropy S of a primary structure subjected to structural optimization after extracting a transmembrane segment and separating and extending the transmembrane segment. As shown in FIG. 14, in Procedures 4 and 5, entropy changes associated with helix formation in Stage 1 of a two-stage model is also taken into consideration. Further in Procedures 1, 3 and 4, the loop structure is also taken into consideration when minimizing a packed structure. Hereinafter, more specific flow examples of Procedures 1 to 5 will be explained in detail.

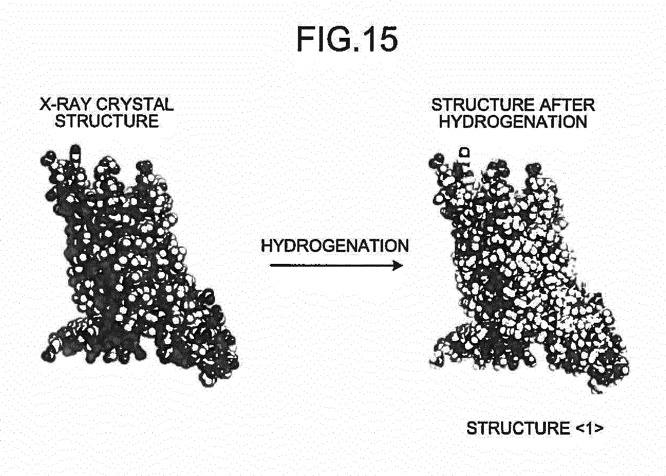

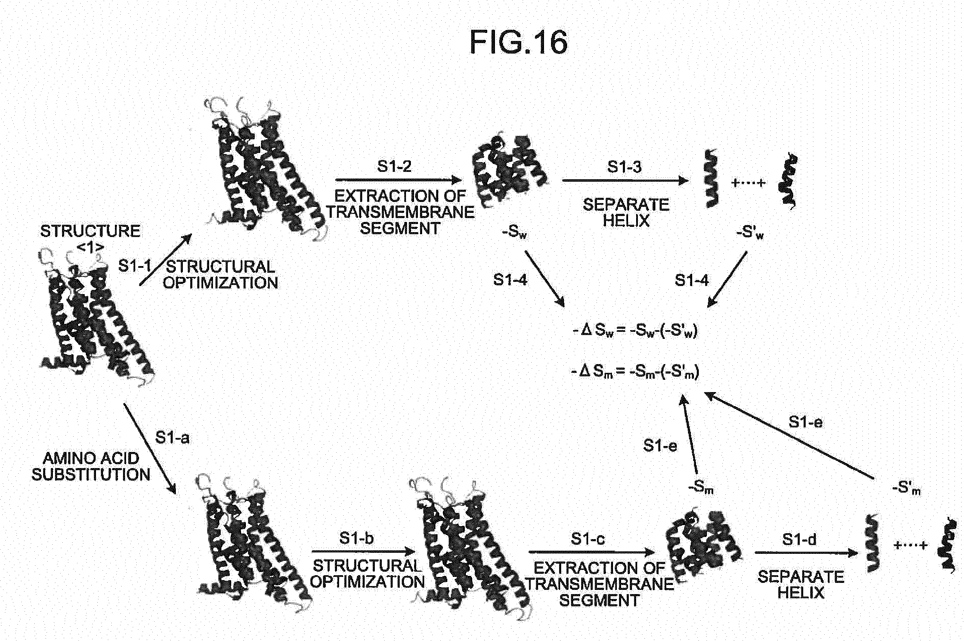

FIG. 15 is a flow diagram showing a common step among Procedures 1 to 5 of the present embodiment. In this example, a crystal structure (PDB code; 3vg9) of a human A2a adenosine receptor was used as the wild type Wt. As a common step among Procedures 1 to 5 explained below in detail, as shown in FIG. 15, the calculating unit 102b hydrogenates the crystal structure of A2aR by using a CHARMM program and an MMTSB program, for example (hereinafter, this structure is referred to as Structure (1)). Hereinafter, the individual flow steps in Procedures 1 to 5 will be explained. FIG. 16 is a flow diagram showing an example of processing in Procedure 1.

Procedure 1

As shown in FIG. 16, first, for Structure (1) of a wild type Wt, the calculating unit 102b uses a CHARMM program to perform structural optimization while relaxing a constraint stepwise, by fixing heavy atoms of the membrane protein and minimizing, fixing $C\alpha$ carbon and $C\beta$ carbon and minimizing, and minimizing without fixation in this order (Step S1-1). Hereinafter, this process is called simply "optimization of structure".

Next, the calculating unit 102b extracts a transmembrane segment alone and calculates its solvation entropy

(-$S_w$) (Step S1-2).

Then, the calculating unit 102b calculates the sum (-$S'_w$) of solvation entropy of structures where their respective helices have been separated (Step S1-3).

Then, the calculating unit 102b calculates the entropy change -$\Delta S_w$=-$S_w$-(-$S'_w$) associated with packing of the helices (Step S1-4).

Meanwhile, for a mutant Mt, the mutation-introducing unit 102a substitutes the amino acid residue of Structure (1) based on the sequence data stored in the sequence file 106b (Step S1-a).

Then, the calculating unit 102b performs structural optimization (Step S1-b).

Then, the calculating unit 102b extracts the transmembrane segment alone and calculates solvation entropy (-$S_m$) (Step S1-c).

Then, the calculating unit 102b calculates the sum (-$S'_m$) of the solvation entropy of the structures where their respective helices have been separated (Step S1-d).

The calculating unit 102b calculates the entropy change -$\Delta S_m$=-$S_m$-(-$S'_m$) associated with packing of the helices (Step S1-e).

From the above-described result, the calculating unit 102b can calculate the entropy change -$\Delta\Delta S$=-$\Delta S_m$-(-$\Delta S_w$) associated with amino acid substitution, which is a difference between the entropy change -$\Delta S_w$ of the wild type Wt and the entropy change -$\Delta S_m$ of the mutant.

Procedure 2

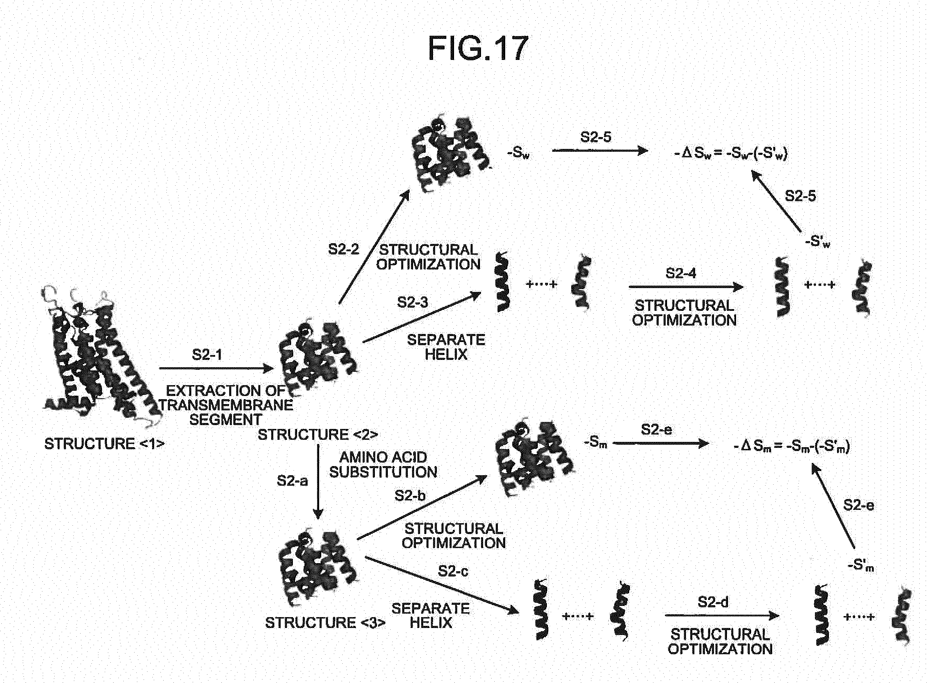

FIG. 17 is a flow diagram showing an example of processing in Procedure 2. As shown in FIG. 17, first, for Structure (1) of a wild type Wt, the calculating unit 102b extracts a transmembrane segment alone (this is referred to as Structure (2)) of Structure (1) (Step S2-1).

Next, the calculating unit 102b performs structural optimization and calculates the solvation entropy (-$S_w$) (Step S2-2).

Then, the calculating unit 102b separates helices of Structure (2) (Step S2-3).

Then, the calculating unit 102b performs optimization of the structures of their respective helices and calculates the sum (-$S'_w$) of the solvation entropy (Step S2-4).

Then, the calculating unit 102b calculates an entropy change -$\Delta S_w$=-$S_w$-(-$S'_w$) associated with packing of the helices (Step S2-5).

Meanwhile, for a mutant Mt, the mutation-introducing unit 102a substitutes the amino acid residue of Structure (2) (this structure is referred to as Structure (3)) (Step S2-a).

Then, the calculating unit 102b performs structural optimization and calculates the solvation entropy (-$S_m$) (Step S2-b) .

Then, the calculating unit 102b separates the helices of Structure (3) (Step S2-c).

Then, the calculating unit 102b performs optimization of the structures of their respective helices, and calculates the sum (-$S'_m$) of the solvation entropy (Step S2-d).

Then, the calculating unit 102b calculates an entropy change -$\Delta S_m$=-$S_m$-(-$S'_m$) associated with packing of the helices (Step S2-e).

From the above-described result, the calculating unit 102b can calculate an entropy change -$\Delta\Delta S$=-$\Delta S_m$-(-$\Delta S_w$) associated with amino acid substitution, which is a difference between the entropy change -$\Delta S_w$ of the wild type Wt and entropy change -$\Delta S_m$ of the mutant.

Procedure 3

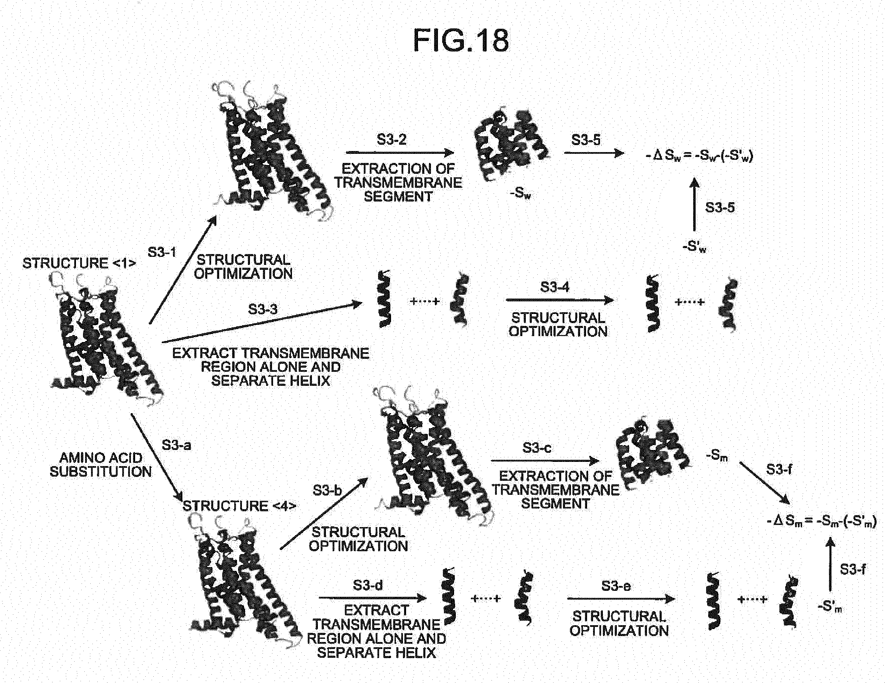

FIG. 18 is a flow diagram showing an example of processing in Procedure 3. As shown in FIG. 18, first, for Structure (1) of the wild type Wt, the calculating unit 102b performs structural optimization of Structure (1) (Step S3-1).

Next, the calculating unit 102b extracts a transmembrane segment alone and calculates the solvation entropy (-$S_w$) (Step S3-2).

Then, the calculating unit 102b extracts a transmembrane segment of Structure (1) and separates helix structures (Step S3-3).

Then, the calculating unit 102b performs optimization of the structures of their respective helices, and calculates the sum (-$S'_w$) of the solvation entropy (Step S3-4).

Then, it calculates an entropy change -$\Delta S_w$=-$S_w$-(-$S'_w$) associated with packing of the helices (Step S3-5).

Meanwhile, for a mutant Mt, the mutation-introducing unit 102a substitutes an amino acid residue of Structure (1) (this structure is referred to as Structure (4)) (Step S3-a).

Then, the calculating unit 102b performs structural optimization of Structure (4) (Step S3-b).

Then, the calculating unit 102b extracts a transmembrane segment alone and calculates the solvation entropy

$(-S_m)$ (Step S3-c) .

Then, the calculating unit 102b extracts a transmembrane segment alone of Structure (4) and separates the helix structures (Step S3-d).

Then, the calculating unit 102b performs optimization of the structures of their respective helices, and calculates the sum $(-S'_m)$ of solvation entropy (Step S3-e) .

Then, the calculating unit 102b calculates an entropy change $-\Delta S_m = -S_m - (-S'_m)$ associated with packing of the helices (Step S3-f).

From the above-described result, the calculating unit 102b can calculate an entropy change $-\Delta\Delta S = -\Delta S_m - (-\Delta S_w)$ associated with amino acid substitution, which is a difference between the entropy change $-\Delta S_w$ of the wild type Wt and the entropy change $-\Delta S_m$ of the mutant.

Procedure 4

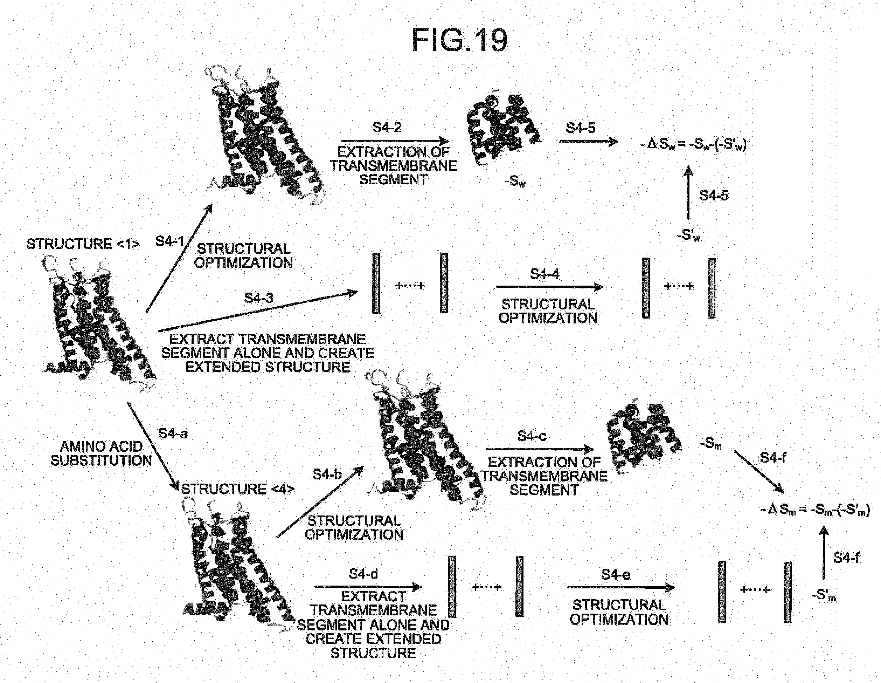

FIG. 19 is a flow diagram showing an example of processing in Procedure 4. As shown in FIG. 19, first, for Structure (1) of a wild type Wt, the calculating unit 102b performs structural optimization of Structure (1) (Step S4-1).

Next, the calculating unit 102b extracts a transmembrane segment alone and calculates the solvation entropy $(-S_w)$ (Step S4-2).

Then, the calculating unit 102b extracts a transmembrane segment of Structure (1), and creates a completely-extended structure (Step S4-3).

Then, the calculating unit 102b performs optimization of their respective extended structures, and calculates the sum $(-S'_w)$ of the solvation entropy (Step S4-4).

Then, the calculating unit 102b calculates an entropy change $-\Delta S_w = -S_w - (-S'_w)$ associated with helix formation and packing from the extended structure (Step S4-5) .

Meanwhile, for a mutant Mt, the mutation-introducing unit 102a substitutes an amino acid residue of Structure (1) (this structure is referred to as Structure (4)) (Step S4-a).

Then, the calculating unit 102b performs structural optimization of Structure (4) (Step S4-b).

Then, the calculating unit 102b extracts a transmembrane segment alone and calculates the solvation entropy $(-S_m)$ (Step S4-c) .

Then, the calculating unit 102b extracts a transmembrane segment alone of Structure (4) and creates a completely-extended structure (Step S4-d).

Then, the calculating unit 102b performs optimization of the respectively-extended structures and calculates the sum $(-S'_m)$ of the solvation entropy (Step S4-e) .

Then, the calculating unit 102b calculates an entropy change $-\Delta S_m = -S_m - (-S'_m)$ associated with helix formation and packing from the extended structures (Step S4-f).

From the result, the calculating unit 102b can calculate an entropy change $-\Delta\Delta S = -\Delta S_m - (-\Delta S_w)$ associated with amino acid substitution, which is a difference between the entropy change $-\Delta S_w$ of the wild type Wt and the entropy change $-\Delta S_m$ of the mutant.

Procedure 5

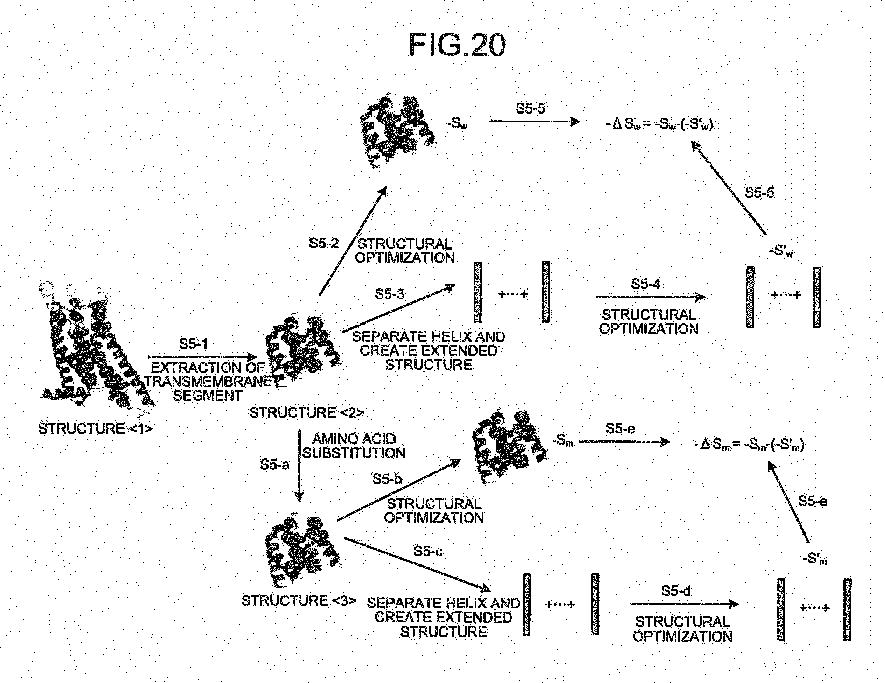

FIG. 20 is a flow diagram showing an example of processing in Procedure 5. As shown in FIG. 20, first, for Structure (1) of a wild type Wt, the calculating unit 102b extracts a transmembrane segment alone (this is referred to as Structure (2)) of Structure (1) (Step S5-1).

Next, the calculating unit 102b performs structural optimization and calculates the solvation entropy $(-S_w)$ (Step S5-2).

Then, the calculating unit 102b separates the helices of Structure (2) and creates a completely-extended structure (Step S5-3).

Then, the calculating unit 102b performs optimization of the respective extended structures and calculates the sum $(-S'_w)$ of the solvation entropy (Step S5-4) .

Then, the calculating unit 102b calculates an entropy change $-\Delta S_w = -S_w - (-S'_w)$ associated with helix and packing from the extended structures (Step S5-5).

Meanwhile, for a mutant Mt, the mutation-introducing unit 102a substitutes an amino acid residue of Structure (2) (Step S5-a).

Then, the calculating unit 102b performs structural optimization of Structure (3) and calculates the solvation entropy $(-S_m)$ (Step S5-b).

Then, the calculating unit 102b separates the helices of Structure (3) and creates completely-extended structures (Step S5-c).

Then, the calculating unit 102b performs optimization of the respective extended structures and calculates the

sum ($-S'_m$) of the solvation entropy (Step S5-d) .

Then, the calculating unit 102b calculates an entropy change $-\Delta S_m = -S_m - (-S'_m)$ associated with helix formation and packing from the extended structures (Step S5-e) .

From the result, the calculating unit 102b can calculate the entropy change $-\Delta\Delta S = -\Delta S_m - (-\Delta S_w)$ associated with amino acid substitution, which is a difference between the entropy change $-\Delta S_w$ of the wild type Wt and the entropy change $-\Delta S_m$ of the mutant.

Comparison with experimental result

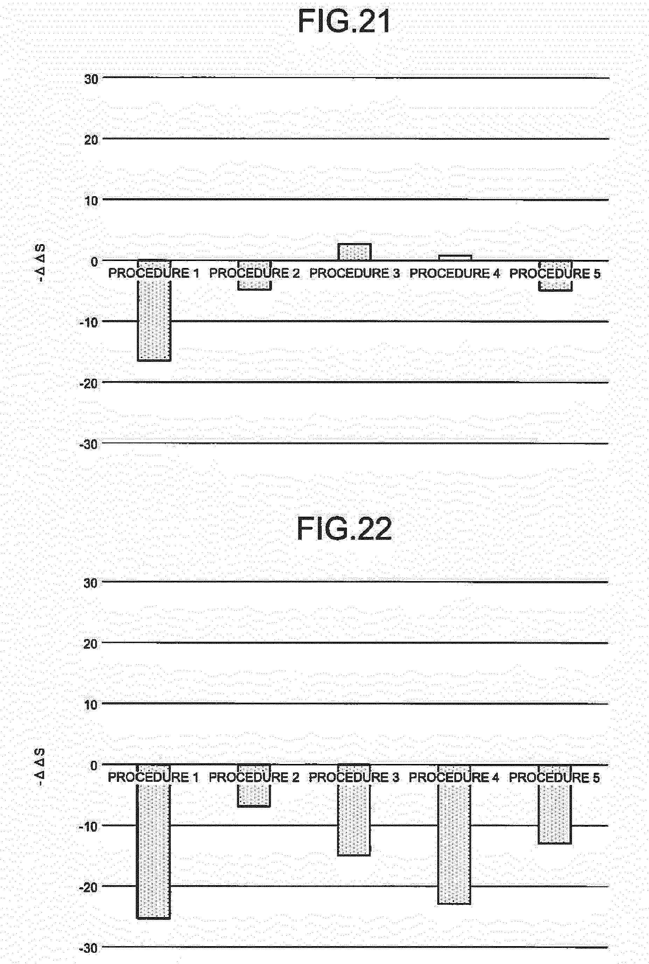

Regarding five kinds of amino acid mutations known to be stabilized or destabilized by substitution, prediction results for thermostabilized mutants by Procedures 1 to 5 were reviewed. FIG. 21 is a diagram showing a calculation result ($-\Delta\Delta S$) by Procedures 1 to 5 for a mutant that is experimentally stabilized, where a threonine residue at position 88 has been substituted by glutamic acid.

As shown in FIG. 21, in Procedures 1, 2 and 5, the value turns to negative and thus the prediction succeeded. FIG. 22 is a diagram showing a calculation result $-\Delta\Delta S$ by Procedures 1 to 5 for a mutant that is experimentally stabilized (namely, its thermal denaturation temperature rises by 8°C), where a serine residue at position 91 has been substituted by arginine.

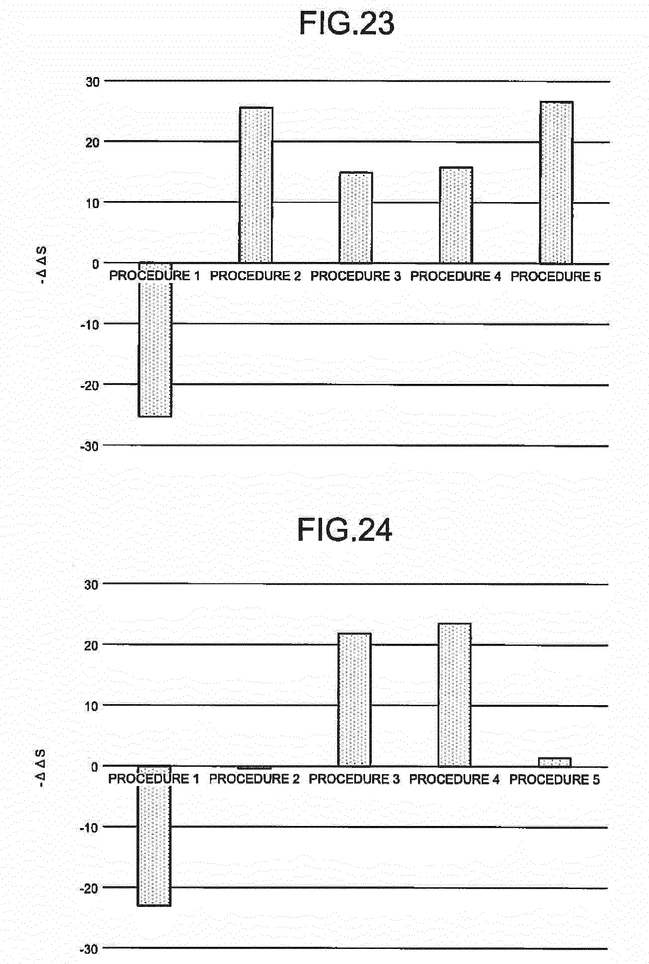

As shown in FIG. 22, all the values turn to negative, and thus the prediction succeeded in all of Procedures. It is expected that stabilization is achieved also by substitution to any other amino acid. FIG. 23 is a diagram showing a calculation result $-\Delta\Delta S$ by Procedures 1 to 5 for a mutant that is experimentally destabilized, where a cysteine residue at position 245 has been substituted by tryptophan.

As shown in FIG. 23, stabilization is predicted in Procedure 1, while destabilization is predicted in the remaining Procedures. FIG. 24 is a diagram showing a calculation result $-\Delta\Delta S$ by Procedures 1 to 5 for a mutant that is experimentally destabilized, where an alanine residue at position 51 has been substituted by tryptophan.

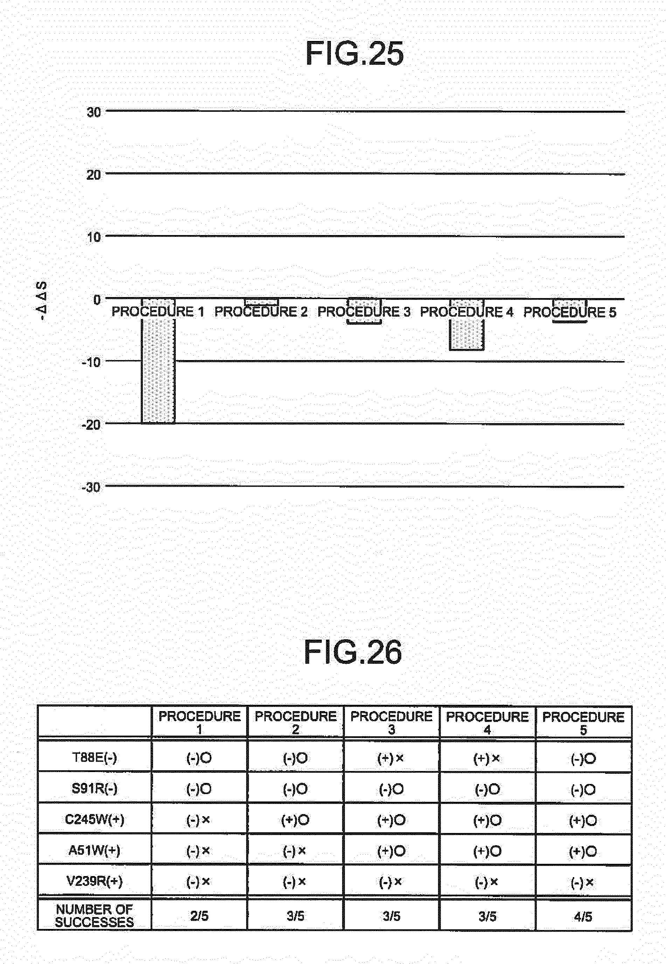

As shown in FIG. 24, stabilization is predicted in Procedure 1. The reason is considered as follows. Due to the substitution, a problem occurs during a process of folding the receptor, and thus a structure predicted to be obtained after the folding cannot be obtained. FIG. 25 is a diagram showing a calculation result $-\Delta\Delta S$ by Procedures 1 to 5 for a mutant that is experimentally destabilized, where a valine residue at position 239 has been substituted by arginine.

As shown in FIG. 25, prediction failed in all of Procedures. FIG. 26 is a table of a prediction result of the thermostabilized mutant by Procedures 1 to 5 for the five kinds of amino acid mutations. Each circle indicates prediction success and each cross mark indicates prediction failure. Each minus sign indicates stabilization and each plus sign indicates destabilization.

As shown in FIG. 26, for the five kinds of amino acid mutations, the prediction success rate by Procedure 5 was high. Further, for example, when Procedures 1 and 2 are combined and a result of stabilization (negative number) is indicated by both of the Procedures, the mutant may be predicted as a stabilized mutant candidate.

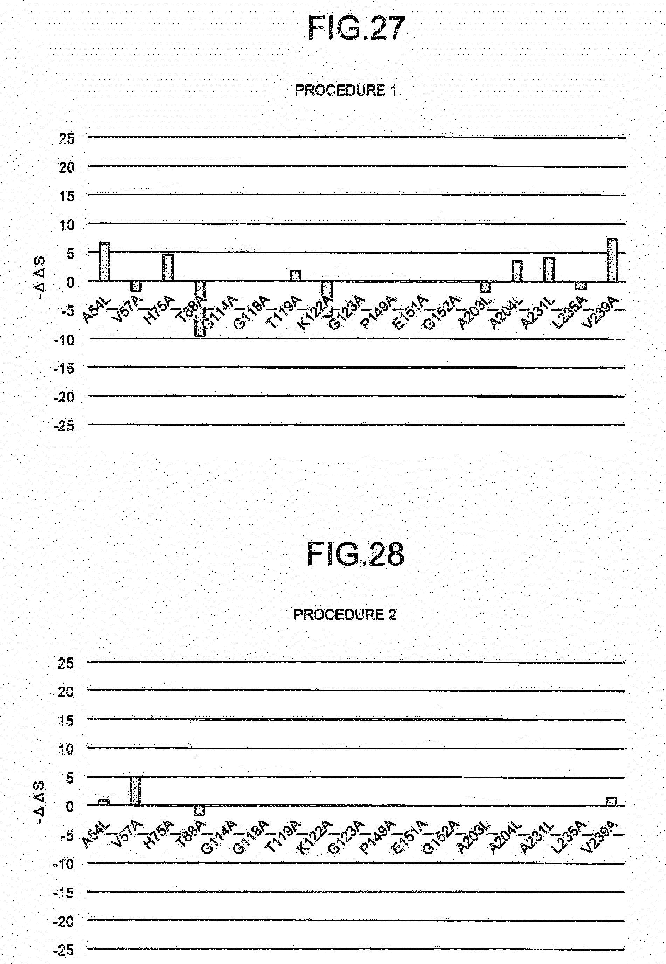

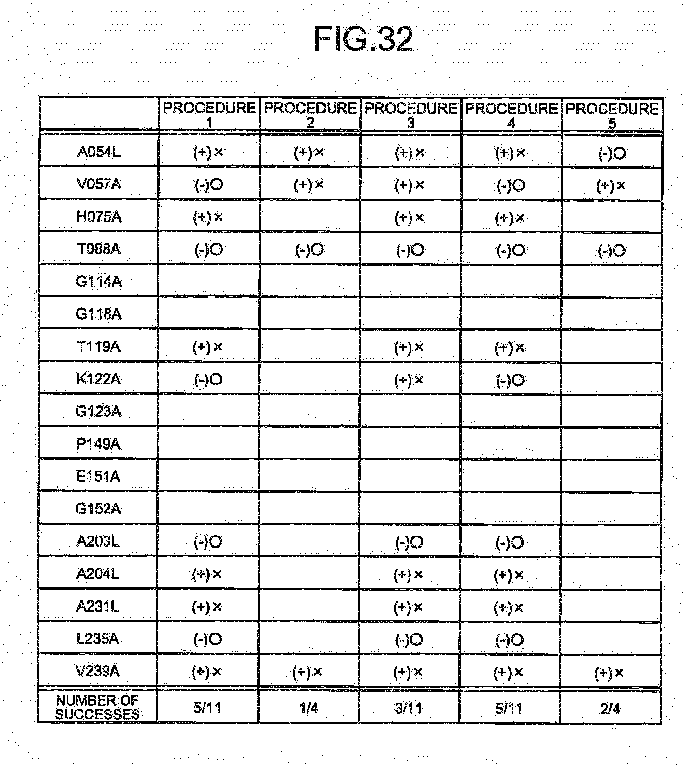

Consequently, the prediction results by Procedures 1 to 5 were reviewed for a group of mutants whose thermal stabilization result had been known due to the experimental results of Tate et al. of the StaR (registered trademark) technique. Tate et al. reports amino acid substitution stabilized at 17 positions from an experimental result by alanine scanning. These results were compared with the $-\Delta\Delta S$ values calculated by Procedures 1 to 5. FIG. 27 to FIG. 31 each is a graph showing a calculation result $-\Delta\Delta S$ in Procedures 1 to 5. FIG. 32 is a table of the prediction results of thermostabilized mutant by Procedures 1 to 5 for the respective amino acid mutations. Each circle indicates prediction success, each cross mark indicates prediction failure, and each blank indicates exclusion from calculation objects. Each minus sign indicates stabilization and each plus sign indicates destabilization. In G114A, G118A, G123A, G152A and the like where glycine has been substituted by alanine, the degree of structural freedom changes considerably, and thus it is considered that influences other than the entropy effect of the membrane are great, namely, the influence of the structural entropy. Therefore, these were excluded from the calculation objects and the columns in the tables were left blank. Similarly, since residues of P149A and E151A are loop portions where crystal structures are not obtained, they were excluded from the calculation objects, and the columns in the tables are left blank. Similarly, since H075A, T119A, K122A, A203L, A204L, A231L and L235A were amino acid residues out of the membrane, they would not be included in the calculation objects in Procedures 2 and 5 where substitution is performed after extracting the transmembrane segment, and thus, the columns for these are left blank.

As shown in FIG. 27 to FIG. 32, use of Procedure 1 or Procedure 4 is considered to be appropriate. The experimental results indicate that T088A is a substitution that improves remarkably the stability, and it is predicted as being stabilized by any calculation of Procedures 1 to 5. Therefore, it is expected that a mutant having a remarkably improved stability can be predicted by selecting substitution to achieve stabilization by any calculation of Procedures 1 to 5.

Example

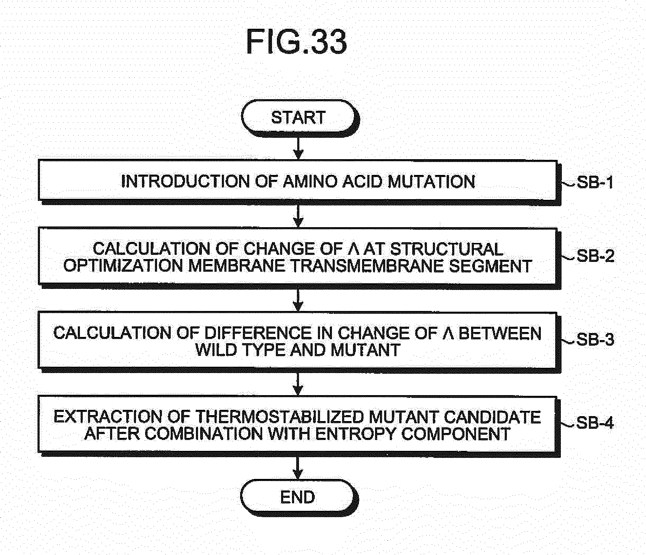

Example of a thermostabilized mutant-predicting apparatus 100 in the present embodiment will be explained with reference to FIG. 1 and FIG. 33 to FIG. 47. FIG. 33 is a flow chart showing an example of processing executed by the thermostabilized mutant-predicting apparatus 100.

As shown in FIG. 33, first, the mutation-introducing unit 102a introduces an amino acid mutation with respect to an amino acid sequence of a membrane protein wild type Wt stored in the sequence file 106b, thereby creating an amino acid sequence of a mutant Mt (Step SB-1). For example, the mutation-introducing unit 102a may create the mutant Mt into which an amino acid mutation such as one amino acid deletion, one amino acid substitution, or one amino acid addition is introduced.

Then, the calculating unit 102b calculates, for the membrane protein wild type Wt and each of the mutants Mt, solvation entropy changes $-\Delta Sw$ and $-\Delta Sm$ in formation of a tertiary structure from a primary structure or formation of the tertiary structure from secondary-structure units within the transmembrane segment involving structural optimization based on the amino acid sequence, and, for the membrane protein wild type Wt and each of the mutants Mt, calculates energy changes $\Delta\Lambda_w$ and $\Delta\Lambda_m$ in formation of the tertiary structure from the primary structure or formation of the tertiary structure from secondary-structure units within the transmembrane segment involving structural optimization based on the amino acid sequence (Step SB-2).

The calculating unit 102b may calculate a change in the solvation entropy by any of Procedures 1 to 5. Further, the calculating unit 102b may calculate the solvation entropy by using integrated methodology of integral equation theory and morphometric representation based on four geometric indices of an excluded volume V, an accessible surface area A, an integrated mean curvature X of accessible surface, and an integrated Gaussian curvature Y of accessible surface.

Regarding the structural optimization, the calculating unit 102b may perform the structural optimization based on not only the amino acid sequence stored in the sequence file 106b but the structural data stored in the structure file 106a. Further, the calculating unit 102b may perform the structural optimization while relaxing a constraint stepwise, by first fixing heavy atoms of the membrane protein and minimizing, then fixing $C\alpha$ carbon and $C\beta$ carbon and minimizing, and finally minimizing without fixation.

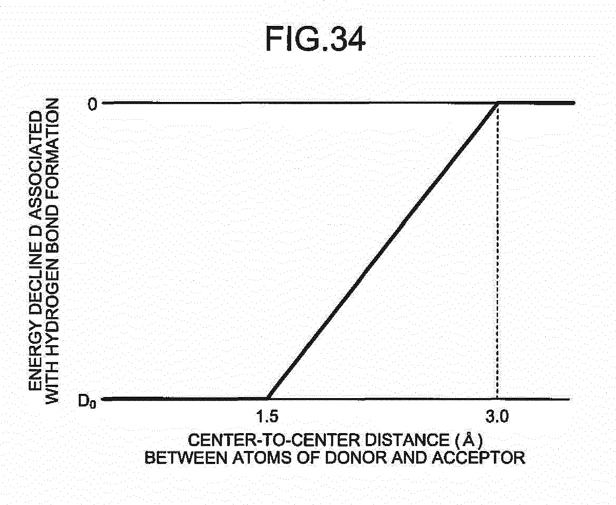

Hereinafter, an example of energy calculation in this Example will be explained with reference to FIG. 1, FIG. 34 and FIG. 35. FIG. 34 is a diagram showing a value of energy lowering at the time of forming one intramolecular hydrogen bond.

In this Example, a free energy function F for a membrane protein is expressed by a formula below.

$$F = -TS + \Lambda$$

(T: absolute temperature, S: entropy component, $\Lambda$: energy component)

The above formula is divided by $k_B T_0$ ($k_B$: Boltzmann's constant, $T_0$=298K) for nondimensionalization, and T is set as $T=T_0$ to provide a formula below.

$$F / (k_B T_0) = -S / k_B + \Lambda / (k_B T_0)$$

As shown in FIG. 1, in this Example, "energy lowering associated with intramolecular hydrogen bond formation=the number of intramolecular hydrogen bonds *D" is calculated based on a completely-extended structure (having no intramolecular hydrogen bond). The D is a value of energy lowering at the time of forming one intramolecular hydrogen bond.

Further as shown in FIG. 34, when the center-to-center distance between atoms of a donor and an acceptor is less than 1.5 Å, D is set to $D_0$ (namely, energy lowering of $D_0$ is given). When the center-to-center distance is equal to or more than 1.5 Å and less than 3.0 Å, D is a value that linearly decreases from 0 to $D_0$ (namely, energy lowering decreased linearly is given), and when the center-to-center distance is equal to or more than 3.0 Å, D is set to be 0 (namely, energy lowering is not given). In this Example, $D_0$ may be $-4k_B T$.

Every donor and acceptor between a main chain and a main chain, between a main chain and a side chain, and between a side chain and a side chain are examined, and for each of which D is calculated and the total sum is taken to calculate "$\Lambda$=energy lowering (negative) associated with intramolecular hydrogen bond formation". It is assumed that acquisition of van der Waals attractive interaction within the protein molecules mutually cancels with loss of the van der Waals attractive interaction between protein and phospholipid.

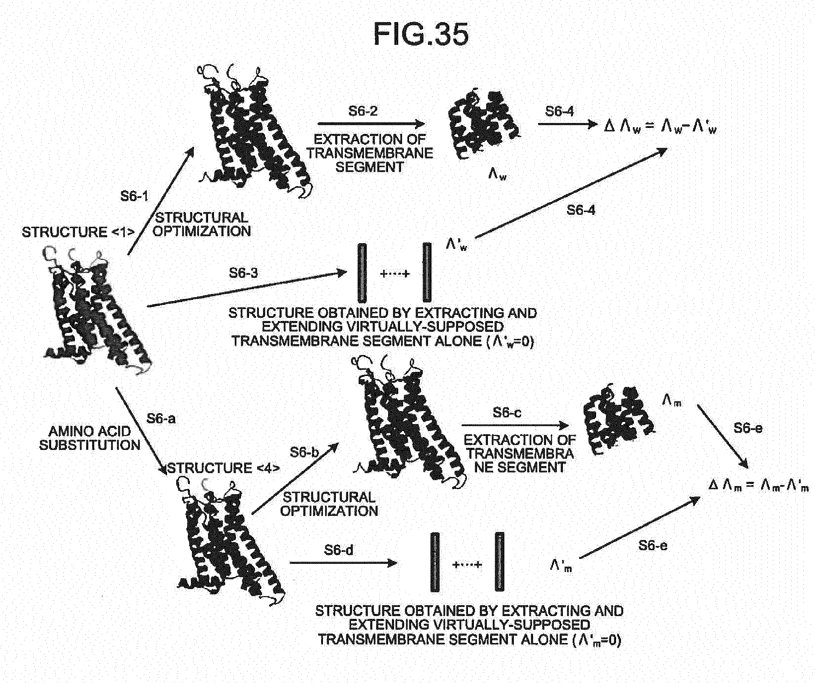

Hereinafter, an example of a step of calculating $\Lambda$ in this Example will be explained with reference to FIG. 35. FIG. 35 is a flow diagram showing an example of processing of $\Lambda$.

As shown in FIG. 35, first, the calculating unit 102b performs structural optimization of Structure (1) of the membrane protein wild type Wt (Step S6-1).

Next, the calculating unit 102b extracts a transmembrane (intercadence) segment alone and calculates the energy ($\Lambda_w$) (Step S6-2).

Then, the calculating unit 102b extracts a transmembrane segment of Structure (1), and acquires a virtually-supposed completely-extended structure ($\Lambda'_w=0$) (Step S6-3).

Then, the calculating unit 102b calculates an energy change $\Delta\Lambda_w=\Lambda_w-\Lambda'_w$, associated with helix formation and packing from the virtually-supposed completely-extended structure ($\Lambda'_w=0$) (Step S6-4).

Meanwhile, for a mutant Mt, the mutation-introducing unit 102a substitutes an amino acid residue of Structure (1) (this structure is regarded as Structure (4)) (Step S6-a).

Then, the calculating unit 102b performs structural optimization of Structure (4) (Step S6-b).

Then, the calculating unit 102b extracts a transmembrane segment alone and calculates the energy ($\Lambda_m$) (Step S6-c).

Then, the calculating unit 102b extracts a transmembrane segment of Structure (4) and acquires a virtually-supposed completely-extended structure ($\Lambda'_m=0$) (Step S6-d).

Then, the calculating unit 102b calculates an energy change $\Delta\Lambda_m=\Lambda_m-\Lambda'_m$ associated with helix formation and packing from the virtually-supposed completely-extended structure ($\Lambda'_m=0$) (Step S6-e).

From the result, the calculating unit 102b can calculate an energy change amount $\Delta\Delta\Lambda=\Delta\Lambda_m-\Delta\Lambda_w$ associated with amino acid substitution of energy lowering caused by folding, which is a difference between the energy change $\Delta\Lambda_w$, of the membrane protein wild type Wt and the energy change $\Delta\Lambda_m$ of the mutant.

Returning to FIG. 33, the candidate-extracting unit 102c calculates a difference ($=-\Delta S_w-(-\Delta S_m)$) between a solvation entropy change $-\Delta S_w$ in the membrane protein wild type and a solvation entropy change $-\Delta S_m$ in the mutant Mt, and calculates a difference $\Delta\Delta\Lambda$ ($=\Delta\Lambda_w-\Delta\Lambda_m$) between an energy change $\Delta\Lambda_w$ in the membrane protein wild type Wt and an energy change $\Delta\Lambda_m$ in the mutant Mt (Step SB-3) .

Then, the candidate-extracting unit 102c extracts a candidate of the mutant Mt to be thermostabilized, based on the sum $\Delta\Delta F$ of the calculated $\Delta\Delta\Lambda$ and $-T\Delta\Delta S$ (Step SB-4). For example, the candidate-extracting unit 102c may determine that the mutant is thermally stabilized when $\Delta\Delta F$ (change amount associated with amino acid substitution of free energy lowering of a system by folding) is a negative value, and that the mutant is thermally destabilized when $\Delta\Delta F$ is a positive value. In an example, the candidate-extracting unit 102c may extract a mutant Mt whose $\Delta\Delta F$ is equal to or less than a predetermined threshold, as a candidate of the mutant Mt to be thermostabilized.

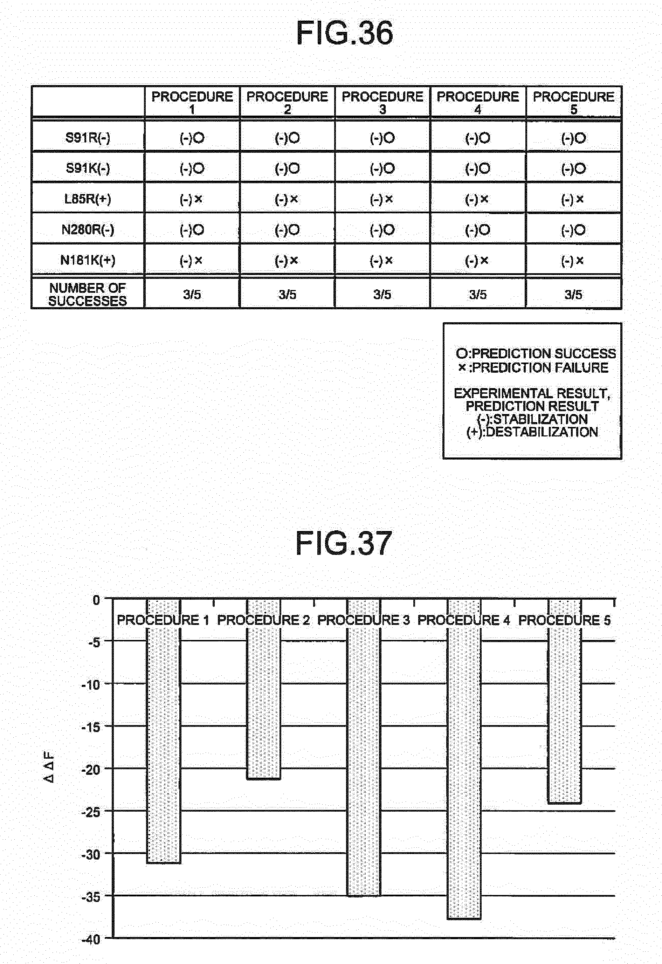

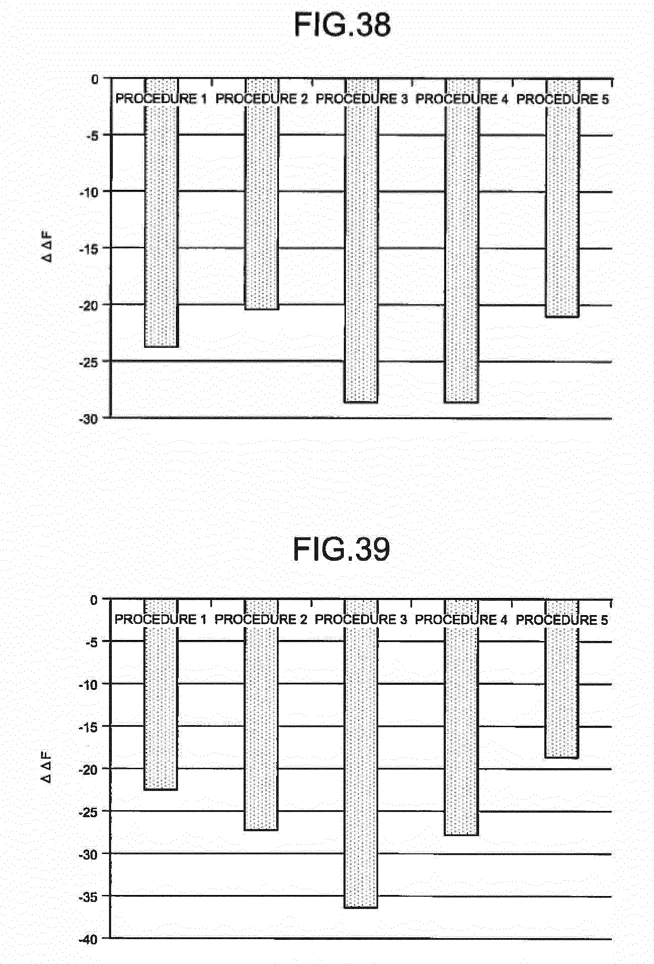

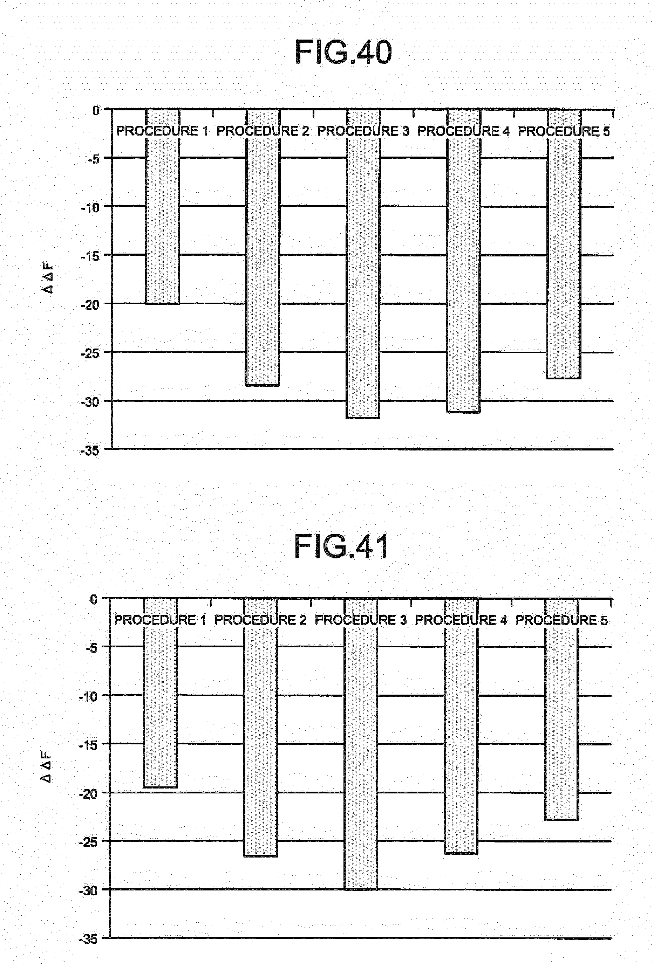

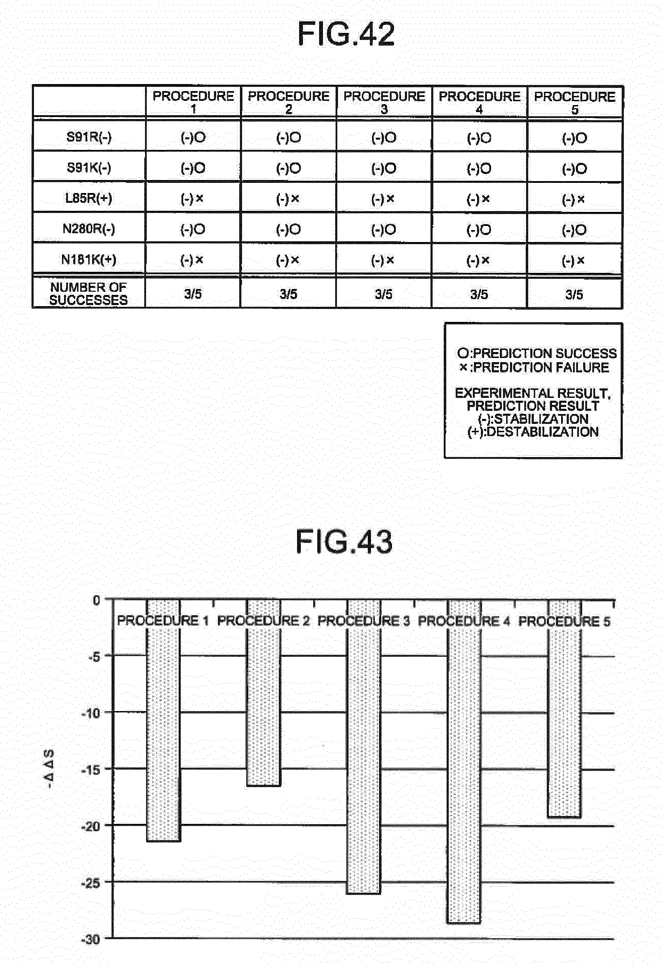

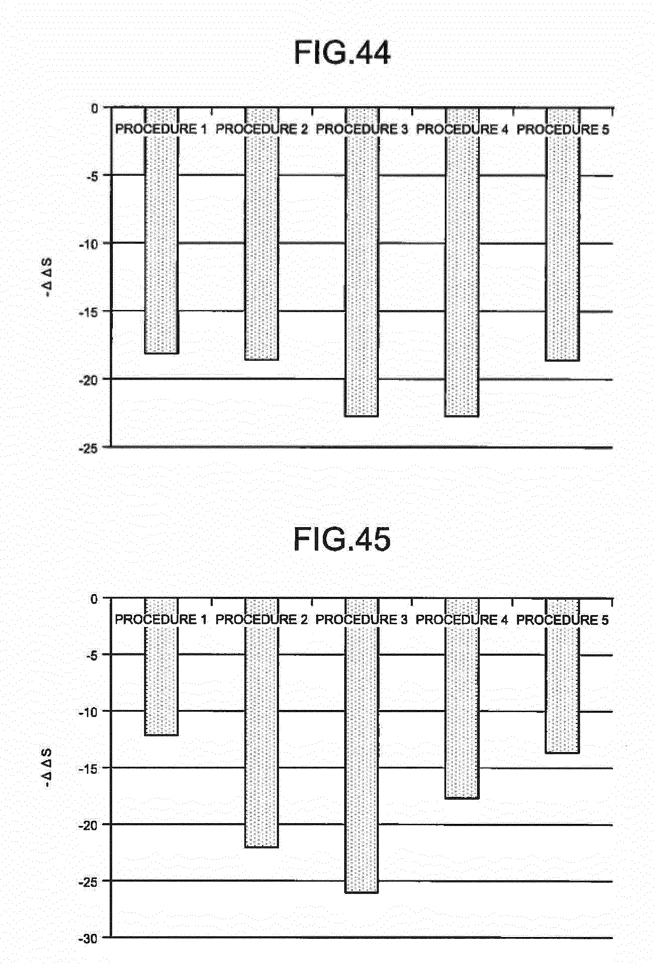

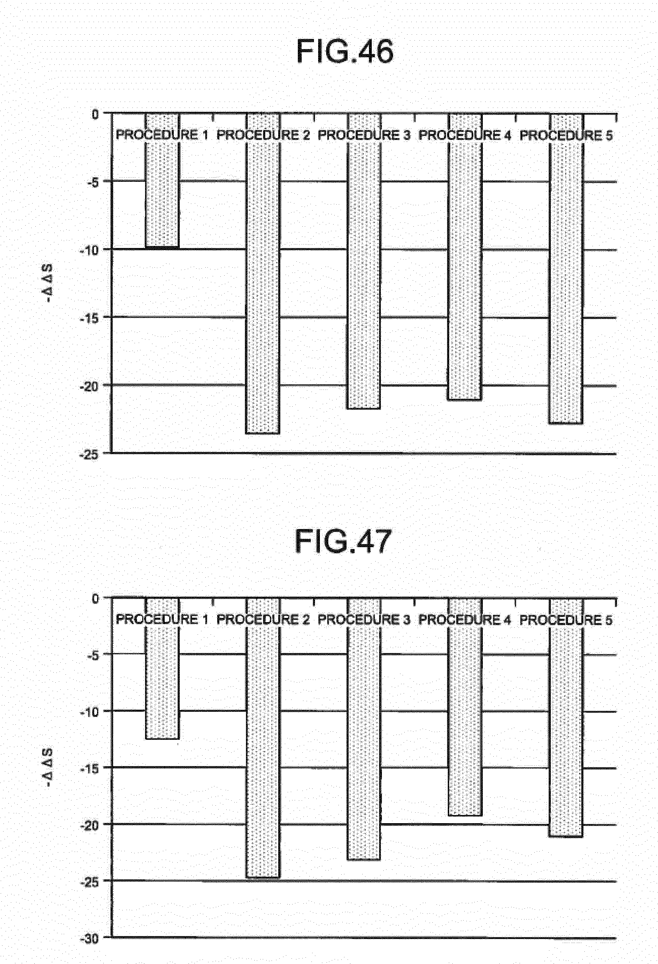

An example of prediction result in the present embodiment will be explained below with reference to FIG. 36 to FIG. 47. FIG. 36 is a diagram showing an example of prediction result. FIG. 37 is a diagram showing values of $\Delta\Delta F$ in Procedures 1 to 5 with respect to S91R. FIG. 38 is a diagram showing values of $\Delta\Delta F$ in Procedures 1 to 5 with respect to S91K. FIG. 39 is a diagram showing values of $\Delta\Delta F$ in Procedures 1 to 5 with respect to L85R. FIG. 40 is a diagram showing values of $\Delta\Delta F$ in Procedures 1 to 5 with respect to N280R. FIG. 41 is a diagram showing values of $\Delta\Delta F$ in Procedures 1 to 5 with respect to N181K. FIG. 42 is a diagram showing an example of prediction result. FIG. 43 is a diagram showing values of in Procedures 1 to 5 with respect to S91R. FIG. 44 is a diagram showing values of in Procedures 1 to 5 with respect to S91K. FIG. 45 is a diagram showing values of in Procedures 1 to 5 with respect to L85R. FIG. 46 is a diagram showing values of in Procedures 1 to 5 with respect to N280R, and FIG. 47 is a diagram showing values of in Procedures 1 to 5 with respect to N181K.

First, among all of the amino acid substitutions performed by using Modeller, five amino acid substitutions (S91R, S91K, L85R, N280R, and N181K) in the order of having smaller $\Delta\Delta F$ value calculated based on Procedure 1 were selected as amino acid substitutions predicted to be stabilized.

Then, as shown in FIG. 36, in this Example, the thermostabilized mutant-predicting method was used to calculate the $\Delta\Delta F$ values with respect to these five amino acid substitutions (FIG. 37 to FIG. 41), and to acquire prediction results (stabilization (-) or destabilization (+)) in a case of using any of Procedure 1 to Procedure 5.

Namely, as shown in FIG. 36, in this Example, irrespective of using any of Procedure 1 to Procedure 5, the values of $\Delta\Delta F$ with respect to these five amino acid substitutions are negative values, and thus they are predicted as mutants to be stabilized (indicated as (-) in the columns of Procedure 1 to Procedure 5 in FIG. 36).

Then, these five amino acid substitutions were subjected to a verification experiment for checking whether they are experimentally stabilized. As shown in FIG. 36, stabilization was confirmed actually in three of these five amino acid substitutions ((-) indicated in each of the left columns in FIG. 36), and thus, a result of prediction success rate of 60% was obtained by using any of Procedure 1 to Procedure 5.

Further, as shown in FIG. 42, in this Example, the thermostabilized mutant-predicting method was used to calculate the values with respect to these five amino acid substitutions (FIG. 43 to FIG. 47), and to acquire a prediction result (stabilization (-) or destabilization (+)) in a case of using any of Procedure 1 to Procedure 5.

Namely, as shown in FIG. 42, in this Example, irrespective of using any of Procedure 1 to Procedure 5, the values of with respect to these five amino acid substitutions are negative values, and thus they are predicted as mutants to be stabilized (indicated as (-) in the columns of Procedure 1 to Procedure 5 in FIG. 42).

Then, these five amino acid substitutions were subjected to a verification experiment for checking whether they are experimentally stabilized. As shown in FIG. 42, stabilization was confirmed actually in three of these five amino acid substitutions ((-) indicated in each of the left columns in FIG. 42), and thus, a result of prediction success rate of 60% was obtained by using any of Procedure 1 to Procedure 5 from the viewpoint limited to entropy.

In this manner, it was clarified that both of the thermostabilized mutant prediction using and the thermostabilized mutant prediction using ΔΔF can provide high prediction success rates in this Example.

Other Embodiments

The present embodiment of the present disclosure has been explained so far. Besides the foregoing embodiment, the present disclosure can also be carried out in various different embodiments within the scope of the technical idea described in the claims.

For example, the thermostabilized mutant-predicting apparatus 100 may perform processing in a standalone mode, or may perform processing according to a request from a client terminal and then return the results of the processing to the client terminal.

Out of the processes explained in relation to the present embodiment, all or some of the processes explained as being automatically performed may be manually performed, or all or some of the processes explained as being manually performed may be automatically performed by publicly known methods.

Besides, the process steps, the control steps, the specific names, the information including registered data for the processes or parameters such as search conditions, the screen examples, or the database configurations described or illustrated herein or the drawings can be appropriately changed if not otherwise specified.

The constituent elements of the thermostabilized mutant-predicting apparatus 100 shown in the drawings are conceptual functions and do not necessarily need to be physically configured as shown in the drawings.

For example, all or any part of the processing functions included in the units of the thermostabilized mutant-predicting apparatus 100, in particular, the processing functions performed by the control unit 102 may be implemented by the CPU or programs interpreted and executed by the CPU, or may be implemented by wired logic-based hardware. The programs including programmed instructions for causing a computer to execute methods according to the present disclosure described later are recorded in non-transitory computer-readable recording media, and are mechanically read by the thermostabilized mutant-predicting apparatus 100 as necessary.
Specifically, the computer programs for giving instructions to the CPU to perform various processes in cooperation with OS are recorded in the storage unit 106 such as a read-only memory (ROM) or a hard disc drive (HDD). The computer programs are loaded into the random access memory (RAM) and executed, and constitute a control unit in cooperation with the CPU.

The computer programs may be stored in an application program server connected to the thermostabilized mutant-predicting apparatus 100 via an appropriate network 300, and may be entirely or partly downloaded as necessary.

The programs according to the present disclosure may be stored in computer-readable recording media or may be formed as program products. The "recording media" include any portable physical media such as a memory card, a USB memory, an SD card, a flexible disc, a magneto optical disc (MO), a ROM, an erasable programmable read only memory (EPROM), an electrically erasable programmable read only memory (EEPROM), a compact disc read only memory (CD-ROM), a digital versatile disc (DVD), and a Blu-ray (registered trademark) disc.

The "programs" constitute data processing methods described in an appropriate language or by an appropriate describing method, and are not limited in format such as source code or binary code. The "programs" are not limited to singly-configured ones but may be distributed into a plurality of modules or libraries or may perform their functions in conjunction with another program typified by an OS. Specific configurations for reading the recording media by the units according to the present embodiment, specific procedures for reading the programs, or specific procedures for installing the read programs may be well-known configurations or procedures.

The various databases and others (structure file 106a, sequence file 106b or the like) stored in the storage unit 106 may be storage units such as any one, some, or all of a memory device such as a RAM or a ROM, a fixed disc device such as a hard disc, a flexible disc, and an optical disc, and may store any one, some, or all of various programs, tables, databases, and web page files for use in various processes and web site provision.

The thermostabilized mutant-predicting apparatus 100 may be an information processing apparatus such as a well-known personal computer, and appropriate peripherals may be connected to the information processing apparatus. The thermostabilized mutant-predicting apparatus 100 may be embodied by providing the information processing apparatus with software (including programs, data, and the like) for implementing the methods according to the present disclosure.

Further, the specific modes of distribution and integration of the devices are not limited to the ones illustrated in the drawings but all or some of the devices may be functionally or physically distributed or integrated by a predetermined unit according to various additions and the like or functional loads. That is, the foregoing embodiments may be carried out in any appropriate combination or may be selectively carried out.

Industrial Applicability

As described above in detail, the present disclosure can provide a thermostabilized mutant-predicting apparatus, a thermostabilized mutant-predicting method and a computer program product capable of suppressing increase in calculation time even when an elongation method is applied to a two-dimensional system or a three-dimensional system, and thus the present disclosure is remarkably useful in various fields such as novel material researches, medical studies, pharmacy, drug discovery, chemical studies, biological studies and clinical examination.

Reference Signs List

| 100 | Thermostabilized mutant-predicting apparatus |
| 102 | Control unit |
| 102a | Mutation-introducing unit |
| 102b | Calculating unit |
| 102c | Candidate-extracting unit |
| 104 | Communication-control interface unit |
| 106 | Storage unit |
| 106a | Structure file |
| 106b | Sequence file |
| 108 | Input/output control interface unit |
| 114 | Input unit |
| 116 | Output unit |
| 200 | External system |
| 300 | Network |

**Claims**

1. A predicting apparatus (100) that predicts a change in solvation entropy of a membrane protein by amino acid substitution , comprising a storage unit (106) and a control unit (102), wherein
the storage unit (106) is configured to store an amino acid sequence of the membrane protein, and
the control unit (102) comprises:

a mutation-introducing unit (102a) configured to introduce an amino acid mutation into the amino acid sequence of the membrane protein to create an amino acid sequence of an amino acid mutant; and
a calculating unit (102b) configured to calculate for each amino acid mutant a solvation entropy change occurring during formation, within a transmembrane segment, of a tertiary structure from a primary structure or from units in a secondary structure;

wherein
the calculating unit (102b) being configured to calculate solvation entropy (S) by the following formula:

$$S/k_B = C_1 V + C_2 A + C_3 X + C_4 Y,$$

where V is the excluded volume, A is the accessible surface area, X is the integrated value of mean curvature of accessible surface and Y is the integrated value of Gaussian curvature of accessible surface,
wherein $C_1$, $C_2$, $C_3$ and $C_4$ are determined by a least square method applied to the following formula:

$$S'/k_B = C_1(4\pi R^3/3) + C_2(4\pi R^2) + C_3(4\pi R) + C_4(4\pi),$$

where R=($d_U$+$d_S$)/2, where $d_S$ is a solvent molecule diameter, and $d_U$ is a spherical (rigid sphere) solute diameter and where S' is the solvation entropy of isolated rigid-sphere solute having various diameters which is calculated by using the integral equation theory.

2. The predicting apparatus (100) according to claim 1, wherein
the calculating unit (102b) is further configured to calculate for the membrane protein a solvation entropy change occurring during formation, within a transmembrane segment, of a tertiary structure from a primary structure or from units of a secondary structure.

3. The predicting apparatus (100) according to any one of claims 1 to 2, wherein
the storage unit (106) is further configured to store structural data of the membrane protein, and
the calculating unit (102b) is configured to perform structural optimization based on the amino acid sequence and the structural data.

4. The predicting apparatus (100) according to any one of claims 1 to 3, wherein
the calculating unit (102b) is configure to perform the structural optimization while relaxing a constraint stepwise, by first fixing heavy atoms of the membrane protein and minimizing, then fixing C$\alpha$ carbon and C$\beta$ carbon and minimizing, and finally minimizing without fixation.

5. The predicting apparatus (100) according to any one of claims 1 to 4, wherein
the calculating unit (102b) is configured to calculate, as the solvation entropy change, a difference between the solvation entropy of the tertiary structure obtained by first structural optimization then extraction of the transmembrane segment, and the solvation entropy of the secondary structure obtained by pulling apart the tertiary structure .

6. The predicting apparatus (100) according to any one of claims 1 to 4, wherein
the calculating unit (102b) is configured to calculate, as the solvation entropy change, a difference between solvation entropy of the tertiary structure obtained by first extraction of the transmembrane segment then structural optimization, and solvation entropy of the secondary structure obtained by first pulling apart the tertiary structure then structural optimization.

7. The predicting apparatus (100) according to any one of claims 1 to 4, wherein
the calculating unit (102b) is configured to calculate, as the solvation entropy change, a difference between solvation entropy of the tertiary structure obtained by first structural optimization then extraction of the transmembrane segment, and solvation entropy of the secondary structure obtained by first extraction of the transmembrane segment then pulling apart transmembrane segment then structural optimization.

8. The predicting apparatus (100) according to any one of claims 1 to 4, wherein
the calculating unit (102b) is configured to calculate, as the solvation entropy change, a difference between solvation entropy of the tertiary structure obtained by first structural optimization then extraction of the transmembrane segment, and solvation entropy of the primary structure obtained by first extraction of the transmembrane segment then stretching the transmembrane segment then structural optimization.

9. The predicting apparatus (100) according to any one of claims 1 to 4, wherein
the calculating unit (102b) is configure to calculate, as the solvation entropy change, a difference between solvation entropy of the tertiary structure obtained by extraction of the transmembrane segment then structural optimization, and solvation entropy of the primary structure obtained by extraction of the transmembrane segment then stretching the transmembrane segment then structural optimization.

10. The predicting apparatus (100) according to any one of claims 1 to 9, wherein
the control unit (102) further comprises:
a candidate-extracting unit (102c) configured to extract a candidate of the amino acid mutant to be thermostabilized, based on a value of the difference between the solvation entropy change in the membrane protein and the solvation entropy changes in the amino acid mutant, an amino acid mutant being extracted as a candidate of the amino acid mutant to be thermo stabilized if the value is a negative value.

11. The predicting apparatus (100) according to claim 10, wherein
the candidate-extracting unit (102c) is configured to extract a candidate of the amino acid mutant to be thermo stabilized, based on a value of the difference between the solvation entropy change in the membrane protein and the solvation entropy change in the amino acid mutant, the amino acid mutant being extracted as a candidate of the amino acid mutant to be thermo stabilized if the value is equal to or less than a certain value.

12. The predicting apparatus (100) according to claim 11 , wherein
the calculating unit (102b) is further configured to calculate for the membrane protein and each amino acid mutant an energy change in formation of the tertiary structure from the primary structure or formation of the tertiary structure from secondary-structure units within the transmembrane segment involving the structural optimization based on the amino acid sequence, and
the candidate-extracting unit (102c) is configured to extract the candidate of the amino acid mutant to be thermostabilized, based on a change amount as a sum of a difference between the energy change in the membrane protein and the energy change in the amino acid mutant, and a value obtained by multiplying an absolute temperature to the difference between the solvation entropy change in the membrane protein and the solvation entropy change in the amino acid mutant.

13. A predicting method for predicting a change in solvation entropy of a membrane protein by amino acid substitution , which is executed in a computer including a storage unit (106) for storing an amino acid sequence of the membrane protein and a control unit (102), comprising:

a mutation-introducing step (SA-1) of introducing an amino acid mutation into the amino acid sequence of the membrane protein to create an amino acid sequence of the amino acid mutant; and
a calculating step (SA-2) of calculating, for each amino acid mutant, a solvation entropy change occurring during formation, within a transmembrane segment, of a tertiary structure from a primary structure or from units of secondary structure, wherein solvation entropy (S) is calculated by the following formula:

$$S/k_B = C_1 V + C_2 A + C_3 X + C_4 Y,$$

where V is the excluded volume, A is the accessible surface area, X is the integrated value of mean curvature of accessible surface and Y is the integrated value of Gaussian curvature of accessible surface,
wherein $C_1$, $C_2$, $C_3$ and $C_4$ are determined by a least square method applied to the following formula:

$$S'/k_B = C_1(4\pi R^3/3) + C_2(4\pi R^2) + C_3(4\pi R) + C_4(4\pi),$$

where $R = (d_U + d_S)/2$, where $d_S$ is a solvent molecule diameter, and $d_U$ is a spherical (rigid sphere) solute diameter and where S' is the solvation entropy of isolated rigid-sphere solute having various diameters which is calculated by using the integral equation theory.

14. The predicting method according to claim 13, wherein
the calculating step (SA-2) comprises calculating, for the membrane protein, a solvation entropy change occuring during formation of a tertiary structure from a primary structure or from units of secondary structure.

15. A computer program product having a non-transitory tangible computer readable medium including programmed instructions for causing a computer including a storage unit (106) for storing an amino acid sequence of a membrane protein and a control unit (102) to perform a predicting method for predicting a change in solvation entropy of a membrane protein by amino acid substitution , comprising:

a mutation-introducing step (SA-1) of introducing an amino acid mutation into the amino acid sequence of the membrane protein to create an amino acid sequence of the amino acid mutant; and
a calculating step (SA-2) of calculating, for each amino acid mutant, a solvation entropy change occurring during formation, within a transmembrane segment, of a tertiary structure from a primary structure or from units of secondary structure wherein solvation entropy (S) is calculated by the following formula:

$$S/k_B = C_1 V + C_2 A + C_3 X + C_4 Y,$$

where V is the excluded volume, A is the accessible surface area, X is the integrated value of mean curvature of accessible surface and Y is the integrated value of Gaussian curvature of accessible surface,
wherein $C_1$, $C_2$, $C_3$ and $C_4$ are determined by a least square method applied to the following formula:

$$S'/k_B = C_1(4\pi R^3/3) + C_2(4\pi R^2) + C_3(4\pi R) + C_4(4\pi),$$

where $R = (d_U + d_S)/2$, where $d_S$ is a solvent molecule diameter, and $d_U$ is a spherical (rigid sphere) solute diameter and where S' is the solvation entropy of isolated rigid-sphere solute having various diameters which is calculated by using the integral equation theory.

16. The computer program product according to claim 15, wherein
the calculating step (SA-2) comprises calculating, for the membrane protein, a solvation entropy change occurring during formation of a tertiary structure from a primary structure or from units of secondary structure.

**Patentansprüche**

1. Vorhersage-Gerät (100), das eine Änderung in der Solvatations-Entropie eines Membranproteins durch Aminosäuresubstitution vorhersagt, umfassend eine Speichereinheit (106) und eine Kontrolleinheit (102), wobei
die Speichereinheit (106) konfiguriert ist, eine Aminosäuresequenz des Membranproteins zu speichern und
die Kontrolleinheit (102) umfasst:
eine Mutations-einführende Einheit (102a), die konfiguriert ist, eine Aminosäuremutation in die Aminosäuresequenz des Membranproteins einzuführen, um eine Aminosäuresequenz einer Aminosäuremutante zu erzeugen; und
eine Berechnungseinheit (102b), die konfiguriert ist, für jede Aminosäuremutante eine Solvatations-Entropieänderung, die während der Bildung, innerhalb eines Transmembransegments, einer Tertiärstruktur aus einer Primärstruktur oder aus Einheiten in einer Sekundärstruktur auftritt, zu berechnen,
wobei
die Berechnungseinheit (102b) konfiguriert ist, die Solvatations-Entropie (S) durch die folgende Formel zu berechnen:

$$S/k_B = C_1 V + C_2 A + C_3 X + C_4 Y,$$

worin V das ausgeschlossene Volumen ist, A der zugängliche Oberflächenbereich ist, X der integrierte Wert der mittleren Krümmung der zugänglichen Oberfläche ist und Y der integrierte Wert der Gaußkrümmung der zugänglichen Oberfläche ist,
wobei $C_1$, $C_2$, $C_3$ und $C_4$ durch ein Verfahren der kleinsten Quadrate berechnet werden, angewendet auf die folgende Formel:

$$S'/k_B = C_1(4\pi R^3/3) + C_2(4\pi R^2) + C_3(4\pi R) + C_4(4\pi),$$

worin $R = (d_U + d_S)/2$, worin $d_S$ ein Durchmesser eines Lösungsmittelmoleküls ist und $d_U$ ein Durchmesser eines sphärischen (feste Sphäre) gelösten Stoffes ist und worin S' die Solvatations-Entropie eines isolierten gelösten Stoffes mit fester Sphäre ist, der verschiedene Durchmesser aufweist, die unter Verwendung der Integralgleichungs-Theorie berechnet wird.

2. Vorhersage-Gerät (100) nach Anspruch 1, wobei
die Berechnungseinheit (102b) weiterhin konfiguriert ist, für das Membranprotein eine Solvatations-Entropieänderung, die während der Bildung, innerhalb eines Transmembransegments, einer Tertiärstruktur aus einer Primärstruktur oder aus Einheiten einer Sekundärstruktur auftritt, zu berechnen.

3. Vorhersage-Gerät (100) nach einem der Ansprüche 1 bis 2, wobei
die Speichereinheit (106) weiterhin konfiguriert ist, Strukturdaten des Membranproteins zu speichern und
die Berechnungseinheit (102b) konfiguriert ist, Strukturoptimierung basierend auf der Aminosäuresequenz und den Strukturdaten durchzuführen.

**4.** Vorhersage-Gerät (100) nach einem der Ansprüche 1 bis 3, wobei die Berechnungseinheit (102b) konfiguriert ist, die Strukturoptimierung durchzuführen, während eine Restriktion schrittweise entspannt wird durch, zuerst, Fixieren schwerer Atome des Membranproteins und Minimieren, dann Fixieren von C$\alpha$-Kohlenstoff und C$\beta$-Kohlenstoff und Minimieren, und schließlich Minimieren ohne Fixierung.

**5.** Vorhersage-Gerät (100) nach einem der Ansprüche 1 bis 4, wobei
die Berechnungseinheit (102b) konfiguriert ist, eine Differenz zwischen der Solvatationsentropie der Tertiärstruktur, die durch zuerst Strukturoptimierung, dann Extraktion des Transmembransegmentes erhalten wurde, und der Solvatationsentropie der Sekundärstruktur, die durch Auseinanderziehen der Tertiärstruktur erhalten wurde, als die Solvatations-Entropieänderung zu berechnen.

**6.** Vorhersage-Gerät (100) nach einem der Ansprüche 1 bis 4, wobei die Berechnungseinheit (102b) konfiguriert ist, eine Differenz zwischen der Solvatationsentropie der Tertiärstruktur, die durch zuerst Extraktion des Transmembransegmentes, dann Strukturoptimierung erhalten wurde, und der Solvatationsentropie der Sekundärstruktur, die durch zuerst Auseinanderziehen der Tertiärstruktur, dann Strukturoptimierung erhalten wurde, als die Solvatations-Entropieänderung zu berechnen.

**7.** Vorhersage-Gerät (100) nach einem der Ansprüche 1 bis 4, wobei die Berechnungseinheit (102b) konfiguriert ist, eine Differenz zwischen der Solvatationsentropie der Tertiärstruktur, die durch zuerst Strukturoptimierung, dann Extraktion des Transmembransegmentes erhalten wurde, und der Solvatationsentropie der Sekundärstruktur, die durch zuerst Extraktion des Transmembransegmentes, dann Auseinanderziehen des Transmembransegmentes, dann Strukturoptimierung erhalten wurde, als die Solvatations-Entropieänderung zu berechnen.

**8.** Vorhersage-Gerät (100) nach einem der Ansprüche 1 bis 4, wobei die Berechnungseinheit (102b) konfiguriert ist, eine Differenz zwischen der Solvatationsentropie der Tertiärstruktur, die durch zuerst Strukturoptimierung, dann Extraktion des Transmembransegmentes erhalten wurde, und der Solvatationsentropie der Primärstruktur, die durch zuerst Extraktion des Transmembransegmentes, dann Spannen des Transmembransegmentes, dann Strukturoptimierung erhalten wurde, als die Solvatations-Entropieänderung zu berechnen.

**9.** Vorhersage-Gerät (100) nach einem der Ansprüche 1 bis 4, wobei die Berechnungseinheit (102b) konfiguriert ist, eine Differenz zwischen der Solvatationsentropie der Tertiärstruktur, die durch Extraktion des Transmembransegmentes, dann Strukturoptimierung erhalten wurde, und der Solvatationsentropie der Primärstruktur, die durch Extraktion des Transmembransegmentes, dann Spannen des Transmembransegmentes, dann Strukturoptimierung erhalten wurde, als die Solvatations-Entropieänderung zu berechnen.

**10.** Vorhersage-Gerät (100) nach einem der Ansprüche 1 bis 9, wobei die Kontrolleinheit (102) weiterhin umfasst:
eine Kandidaten-extrahierende Einheit (102c), die konfiguriert ist, einen Kandidaten der Aminosäuremutante, die thermostabilisiert werden soll, zu extrahieren, basierend auf einem Wert der Differenz zwischen der Solvatations-Entropieänderung in dem Membranprotein und den Solvatations-Entropieänderungen in der Aminosäuremutante, wobei eine Aminosäuremutante als ein Kandidat der Aminosäuremutante, die thermostabilisiert werden soll, extrahiert wird, wenn der Wert ein negativer Wert ist.

**11.** Vorhersage-Gerät (100) nach Anspruch 10, wobei
die Kandidaten-extrahierende Einheit (102c) konfiguriert ist, einen Kandidaten der Aminosäuremutante, die thermostabilisiert werden soll, zu extrahieren, basierend auf einem Wert der Differenz zwischen der Solvatations-Entropieänderung in dem Membranprotein und der Solvatations-Entropieänderung in der Aminosäuremutante, wobei die Aminosäuremutante als ein Kandidat der Aminosäuremutante, die thermostabilisiert werden soll, extrahiert wird, wenn der Wert gleich zu oder niedriger als ein bestimmter Wert ist.

**12.** Vorhersage-Gerät (100) nach Anspruch 11, wobei
die Berechnungseinheit (102b) weiterhin konfiguriert ist, für das Membranprotein und jede Aminosäuremutante eine Energieänderung bei der Bildung der Tertiärstruktur aus der Primärstruktur oder Bildung der Tertiärstruktur aus Sekundärstruktureinheiten innerhalb des Transmembransegments unter Einbeziehung der Strukturoptimierung basierend auf der Aminosäuresequenz zu berechnen, und
die Kandidaten-extrahierende Einheit (102c) konfiguriert ist, den Kandidaten der Aminosäuremutante, die thermostabilisiert werden soll, zu extrahieren, basierend auf einer Änderungsmenge als eine Summe einer Differenz zwischen der Energieänderung in dem Membranprotein und der Energieänderung in der Aminosäuremutante, und einem Wert, der durch Multiplizieren einer absoluten Temperatur zur Differenz zwischen der Solvatations-Entropieänderung in dem Membranprotein und der Solvatations-Entropieänderung in der Aminosäuremutante erhalten wird.

13. Vorhersageverfahren zur Vorhersage einer Änderung in der Solvatations-Entropie eines Membranproteins durch Aminosäuresubstitution, welches in einem Computer ausgeführt wird, der eine Speichereinheit (106) zum Speichern einer Aminosäuresequenz des Membranproteins und eine Kontrolleinheit (102) umfasst, umfassend:

einen Mutations-Einführungsschritt (SA-1) zum Einführen einer Aminosäuremutation in die Aminosäuresequenz des Membranproteins, um eine Aminosäuresequenz der Aminosäuremutante zu erzeugen; und
einen Berechnungsschritt (SA-2) zum Berechnen, für jede Aminosäuremutante, einer Solvatations-Entropieänderung, die während der Bildung, innerhalb eines Transmembransegments, einer Tertiärstruktur aus einer Primärstruktur oder aus Einheiten einer Sekundärstruktur auftritt,

wobei die Solvatations-Entropie (S) durch die folgende Formel berechnet wird:

$$S/k_B = C_1V + C_2A + C_3X + C_4Y,$$

worin V das ausgeschlossene Volumen ist, A der zugängliche Oberflächenbereich ist, X der integrierte Wert der mittleren Krümmung der zugänglichen Oberfläche ist und Y der integrierte Wert der Gaußkrümmung der zugänglichen Oberfläche ist,
wobei $C_1$, $C_2$, $C_3$ und $C_4$ durch ein Verfahren der kleinsten Quadrate berechnet werden, angewendet auf die folgende Formel:

$$S'/k_B = C_1(4\pi R^3/3) + C_2(4\pi R^2) + C_3(4\pi R) + C_4(4\pi),$$

worin R = $(d_U + d_S)/2$, worin $d_S$ ein Durchmesser eines Lösungsmittelmoleküls ist und $d_U$ ein Durchmesser eines sphärischen (feste Sphäre) gelösten Stoffes ist und worin S' die Solvatations-Entropie eines isolierten gelösten Stoffes mit fester Sphäre ist, der verschiedene Durchmesser aufweist, die unter Verwendung der Integralgleichungs-Theorie berechnet wird.

14. Vorhersageverfahren nach Anspruch 13, wobei
der Berechnungsschritt (SA-2) Berechnen, für das Membranprotein, einer Solvatations-Entropieänderung, die während der Bildung einer Tertiärstruktur aus einer Primärstruktur oder aus Einheiten einer Sekundärstruktur auftritt, umfasst.

15. Computerprogrammprodukt mit einem nicht-transitorischen greifbaren computerlesbaren Medium, das programmierte Anweisungen umfasst, um einen Computer, der eine Speichereinheit (106) zum Speichern einer Aminosäuresequenz eines Membranproteins und eine Kontrolleinheit (102) umfasst, zu veranlassen, ein Vorhersageverfahren zur Vorhersage einer Änderung in der Solvatations-Entropie eines Membranproteins durch Aminosäuresubstitution durchzuführen, umfassend:

einen Mutations-Einführungsschritt (SA-1) zum Einführen einer Aminosäuremutation in die Aminosäuresequenz des Membranproteins, um eine Aminosäuresequenz der Aminosäuremutante zu erzeugen; und
einen Berechnungsschritt (SA-2) zum Berechnen, für jede Aminosäuremutante, einer Solvatations-Entropieänderung, die während der Bildung, innerhalb eines Transmembransegments, einer Tertiärstruktur aus einer Primärstruktur oder aus Einheiten einer Sekundärstruktur auftritt,

wobei die Solvatations-Entropie (S) durch die folgende Formel berechnet wird:

$$S/k_B = C_1V + C_2A + C_3X + C_4Y,$$

worin V das ausgeschlossene Volumen ist, A der zugängliche Oberflächenbereich ist, X der integrierte Wert der mittleren Krümmung der zugänglichen Oberfläche ist und Y der integrierte Wert der Gaußkrümmung der zugänglichen Oberfläche ist,
wobei $C_1$, $C_2$, $C_3$ und $C_4$ durch ein Verfahren der kleinsten Quadrate berechnet werden, angewendet auf die folgende Formel:

$$S'/k_B = C_1(4\pi R^3/3) + C_2(4\pi R^2) + C_3(4\pi R) + C_4(4\pi),$$

worin R = $(d_U + d_S)/2$, worin $d_S$ ein Durchmesser eines Lösungsmittelmoleküls ist und $d_U$ ein Durchmesser eines sphärischen (feste Sphäre) gelösten Stoffes ist und worin S' die Solvatations-Entropie eines isolierten gelösten Stoffes mit fester Sphäre ist, der verschiedene Durchmesser aufweist, die unter Verwendung der Integralgleichungs-Theorie berechnet wird.

16. Computerprogrammprodukt nach Anspruch 15, wobei
der Berechnungsschritt (SA-2) Berechnen, für das Membranprotein, einer Solvatations-Entropieänderung, die während der Bildung einer Tertiärstruktur aus einer Primärstruktur oder aus Einheiten einer Sekundärstruktur auftritt, umfasst.

**Revendications**

1. Appareil de prédiction (100) qui prédit une modification de l'entropie de solvatation d'une protéine membranaire par substitution d'acides aminés, comprenant une unité de stockage (106) et une unité de commande (102), dans lequel l'unité de stockage (106) est configurée pour stocker une séquence d'acides aminés de la protéine membranaire, et l'unité de commande (102) comprend :

   une unité d'introduction de mutation (102a) configurée pour introduire une mutation d'acides aminés dans la séquence d'acides aminés de la protéine membranaire pour créer une séquence d'acides aminés d'un mutant d'acides aminés ; et
   une unité de calcul (102b) configurée pour calculer pour chaque mutant d'acides aminés une modification d'entropie de solvatation survenant durant la formation, à l'intérieur d'un segment transmembranaire, d'une structure tertiaire à partir d'une structure primaire ou à partir d'unités dans une structure secondaire ;
   dans lequel
   l'unité de calcul (102b) étant configurée pour calculer une entropie de solvatation (S) par la formule suivante :

$$S/k_B = C_1 V + C_2 A + C_3 X + C_4 Y,$$

   où V est le volume exclu, A est l'aire de surface accessible, X est la valeur intégrée de la courbure moyenne de la surface accessible et Y est la valeur intégrée de la courbure gaussienne de la surface accessible, dans lequel $C_1$, $C_2$, $C_3$ et $C_4$ sont déterminés par la méthode des moindres carrés appliquée à la formule suivante :

$$S'/k_B = C_1(4\pi R^3/3) + C_2(4\pi R^2) + C_3(4\pi R) + C_4(4\pi),$$

   où R = $(d_u + d_s)/2$, où $d_s$ est un diamètre de molécule de solvant, et $d_u$ est un diamètre de soluté sphérique (sphère rigide) et où S' est l'entropie de solvatation de soluté de sphère rigide isolé ayant différents diamètres qui est calculée en utilisant la théorie de l'équation intégrale.

2. Appareil de prédiction (100) selon la revendication 1, dans lequel
l'unité de calcul (102b) est en outre configurée pour calculer pour la protéine membranaire une modification d'entropie de solvatation survenant durant la formation, à l'intérieur d'un segment transmembranaire, d'une structure tertiaire à partir d'une structure primaire ou à partir d'unités d'une structure secondaire.

3. Appareil de prédiction (100) selon l'une quelconque des revendications 1 à 2, dans lequel
l'unité de stockage (106) est en outre configurée pour stocker les données structurales de la protéine membranaire, et l'unité de calcul (102b) est configurée pour réaliser l'optimisation structurale sur la base de la séquence d'acides aminés et des données structurales.

4. Appareil de prédiction (100) selon l'une quelconque des revendications 1 à 3, dans lequel

l'unité de calcul (102b) est configurée pour réaliser l'optimisation structurale tout en relâchant une contrainte graduellement, d'abord par fixation des atomes lourds de la protéine membranaire et minimisation, puis par fixation du carbone C$\alpha$ et du carbone C$\beta$ et minimisation, et finalement minimisation sans fixation.

5. Appareil de prédiction (100) selon l'une quelconque des revendications 1 à 4, dans lequel l'unité de calcul (102b) est configurée pour calculer, en tant que modification de l'entropie de solvatation, une différence entre l'entropie de solvatation de la structure tertiaire obtenue d'abord par optimisation structurale puis par extraction du segment transmembranaire, et l'entropie de solvatation de la structure secondaire obtenue d'abord par démantèlement de la structure tertiaire.

6. Appareil de prédiction (100) selon l'une quelconque des revendications 1 à 4, dans lequel l'unité de calcul (102b) est configurée pour calculer, en tant que modification de l'entropie de solvatation, une différence entre l'entropie de solvatation de la structure tertiaire obtenue d'abord par extraction du segment transmembranaire puis optimisation structurale, et l'entropie de solvatation de la structure secondaire obtenue d'abord par démantèlement de la structure tertiaire puis optimisation structurale.

7. Appareil de prédiction (100) selon l'une quelconque des revendications 1 à 4, dans lequel l'unité de calcul (102b) est configurée pour calculer, en tant que modification de l'entropie de solvatation, une différence entre l'entropie de solvatation de la structure tertiaire obtenue d'abord par optimisation structurale puis par extraction du segment transmembranaire, et l'entropie de solvatation de la structure secondaire obtenue d'abord par extraction du segment transmembranaire puis démantèlement du segment transmembranaire puis optimisation structurale.

8. Appareil de prédiction (100) selon l'une quelconque des revendications 1 à 4, dans lequel l'unité de calcul (102b) est configurée pour calculer, en tant que modification de l'entropie de solvatation, une différence entre l'entropie de solvatation de la structure tertiaire obtenue d'abord par optimisation structurale puis par extraction du segment transmembranaire, et l'entropie de solvatation de la structure primaire obtenue d'abord par extraction du segment transmembranaire puis étirement du segment transmembranaire puis optimisation structurale.

9. Appareil de prédiction (100) selon l'une quelconque des revendications 1 à 4, dans lequel l'unité de calcul (102b) est configurée pour calculer, en tant que modification de l'entropie de solvatation, une différence entre l'entropie de solvatation de la structure tertiaire obtenue par extraction du segment transmembranaire puis optimisation structurale, et l'entropie de solvatation de la structure primaire obtenue par extraction du segment transmembranaire puis étirement du segment transmembranaire puis optimisation structurale.

10. Appareil de prédiction (100) selon l'une quelconque des revendications 1 à 9, dans lequel l'unité de commande (102) comprend en outre :
une unité d'extraction de candidat (102c) configurée pour extraire un candidat du mutant d'acides aminés à thermostabiliser, sur la base d'une valeur de la différence entre la modification d'entropie de solvatation dans la protéine membranaire et les modifications d'entropie de solvatation dans le mutant d'acides aminés, un mutant d'acides aminés étant extrait en tant que candidat du mutant d'acides aminés à thermostabiliser si la valeur est une valeur négative.

11. Appareil de prédiction (100) selon la revendication 10, dans lequel l'unité d'extraction de candidat (102c) est configurée pour extraire un candidat du mutant d'acides aminés à thermostabiliser, sur la base d'une valeur de la différence entre la modification d'entropie de solvatation dans la protéine membranaire et la modification d'entropie de solvatation dans le mutant d'acides aminés, le mutant d'acides aminés étant extrait en tant que candidat du mutant d'acides aminés à thermostabiliser si la valeur est inférieure ou égale à une certaine valeur.

12. Appareil de prédiction (100) selon la revendication 11, dans lequel l'unité de calcul (102b) est en outre configurée pour calculer pour la protéine membranaire et chaque mutant d'acides aminés une modification d'énergie dans la formation de la structure tertiaire à partir de la structure primaire ou la formation de la structure tertiaire à partir d'unités de structure secondaire à l'intérieur du segment transmembranaire impliquant l'optimisation structurale sur la base de la séquence d'acides aminés, et l'unité d'extraction de candidat (102c) est configurée pour extraire le candidat du mutant d'acides aminés à thermostabiliser, sur la base d'une quantité modifiée en tant que somme d'une différence entre la modification d'énergie dans la protéine membranaire et la modification d'énergie dans le mutant d'acides aminés, et d'une valeur obtenue par multiplication d'une température absolue jusqu'à la différence entre la modification d'entropie de solvatation dans la protéine membranaire et la modification d'entropie de solvatation dans le mutant d'acides aminés.

13. Procédé de prédiction d'une modification de l'entropie de solvatation d'une protéine membranaire par substitution d'acides aminés, qui est exécuté dans un ordinateur incluant une unité de stockage (106) pour stocker la séquence d'acides aminés de la protéine membranaire et une unité de commande (102), comprenant :

une étape d'introduction de mutation (SA-1) consistant à introduire une mutation d'acides aminés dans la séquence d'acides aminés de la protéine membranaire pour créer une séquence d'acides aminés du mutant d'acides aminés ;
une étape de calcul (SA-2) consistant à calculer, pour chaque mutant d'acides aminés, une modification d'entropie de solvatation survenant durant la formation, à l'intérieur d'un segment transmembranaire, d'une structure tertiaire à partir d'une structure primaire ou à partir d'unités de structure secondaire, dans lequel l'entropie de solvatation (S) est calculée par la formule suivante :

$$S/k_B = C_1 V + C_2 A + C_3 X + C_4 Y,$$

où V est le volume exclu, A est l'aire de surface accessible, X est la valeur intégrée de la courbure moyenne de la surface accessible et Y est la valeur intégrée de la courbure gaussienne de la surface accessible, dans lequel $C_1$, $C_2$, $C_3$ et $C_4$ sont déterminés par la méthode des moindres carrés appliquée à la formule suivante :

$$S'/k_B = C_1(4\pi R^3/3) + C_2(4\pi R^2) + C_3(4\pi R) + C_4(4\pi),$$

où $R = (d_u + d_s)/2$, où $d_s$ est un diamètre de molécule de solvant, et $d_u$ est un diamètre de soluté sphérique (sphère rigide) et où S' est l'entropie de solvatation de soluté de sphère rigide isolé ayant différents diamètres qui est calculée en utilisant la théorie de l'équation intégrale.

14. Procédé de prédiction selon la revendication 13, dans lequel
l'étape de calcul (SA-2) comprend le calcul, pour la protéine membranaire, d'une modification de l'entropie de solvatation survenant durant la formation d'une structure tertiaire à partir d'une structure primaire ou à partir d'unités de structure secondaire.

15. Produit de programme informatique ayant un support lisible par ordinateur tangible non transitoire incluant des instructions programmées pour amener un ordinateur incluant une unité de stockage (106) pour stocker une séquence d'acides aminés de la protéine membranaire et une unité de commande (102) à réaliser un procédé de prédiction pour prédire une modification de l'entropie de solvatation d'une protéine membranaire par substitution d'acides aminés, comprenant :

une étape d'introduction de mutation (SA-1) consistant à introduire une mutation d'acides aminés dans la séquence d'acides aminés de la protéine membranaire pour créer une séquence d'acides aminés du mutant d'acides aminés ; et
une étape de calcul (SA-2) consistant à calculer, pour chaque mutant d'acides aminés, une modification d'entropie de solvatation survenant durant la formation, à l'intérieur d'un segment transmembranaire, d'une structure tertiaire à partir d'une structure primaire ou à partir d'unités de structure secondaire, dans lequel l'entropie de solvatation (S) est calculée par la formule suivante :

$$S/k_B = C_1 V + C_2 A + C_3 X + C_4 Y,$$

où V est le volume exclu, A est l'aire de surface accessible, X est la valeur intégrée de la courbure moyenne de la surface accessible et Y est la valeur intégrée de la courbure gaussienne de la surface accessible, dans lequel $C_1$, $C_2$, $C_3$ et $C_4$ sont déterminés par la méthode des moindres carrés appliquée à la formule suivante :

$$S'/k_B=C_1(4\pi R^3/3)+C_2(4\pi R^2)+C_3(4\pi R)+C_4(4\pi),$$

où $R = (d_u + d_s)/2$, où $d_s$ est un diamètre de molécule de solvant, et $d_u$ est un diamètre de soluté sphérique (sphère rigide) et où S' est l'entropie de solvatation de soluté de sphère rigide isolé ayant différents diamètres qui est calculée en utilisant la théorie de l'équation intégrale.

16. Produit de programme informatique selon la revendication 15, dans lequel
l'étape de calcul (SA-2) comprend le calcul, pour la protéine membranaire, d'une modification d'entropie de solvatation durant la formation d'une structure tertiaire à partir d'une structure primaire ou à partir d'unités de structure secondaire.

# FIG.1

EXPOSURE [ NH ] + [ CO ] $\xrightarrow{\text{D}}$ INTRAMOLECULAR HYDROGEN BOND [ CO···HN ]

$\downarrow 0$  $\downarrow 0$  $\searrow$ D  $\downarrow 0$

BURYING [ NH ] + [ CO ] $\xrightarrow{\text{D}}$ [ CO···HN ]

INTRAMOLECULAR HYDROGEN BOND

# FIG.2

EXPOSURE [ NH ] + [ O ] $\xrightarrow{-14k_BT_0}$ INTRAMOLECULAR HYDROGEN BOND [ O···HN ]

$\downarrow 0$  $\downarrow 0$  $\searrow -14k_BT_0$  $\downarrow 0$

BURYING [ NH ] + [ O ] $\xrightarrow{-14k_BT_0}$ [ O···HN ]

INTRAMOLECULAR HYDROGEN BOND

# FIG.3

# FIG.4

# FIG.5

# FIG.6

NS
(NATIVE STRUCTURE)

15000 FALSE STRUCTURES
(GENERATED BY REPLICA EXCHANGE MONTE CARLO SIMULATION)

EP 3 163 481 B1

# FIG.7

NS AND 15000 FALSE STRUCTURES

# FIG.8

# FIG.9

# FIG.10

INPUT UNIT ⌐114

OUTPUT UNIT ⌐116

⌐100

THERMOSTABILIZED
MUTANT-PREDICTING
APPARATUS

⌐108
INPUT/OUTPUT CONTROL
INTERFACE
UNIT

⌐106
STORAGE UNIT

⌐102
CONTROL UNIT

104

⌐200
EXTERNAL SYSTEM

⌐106a
STRUCTURE FILE

⌐102a
MUTATION-
INTRODUCING UNIT

⌐300

⌐106b
SEQUENCE FILE

⌐102b
CALCULATING
UNIT

COMMUNICATION-CONTROL
INTERFACE UNIT

⌐102c
CANDIDATE-
EXTRACTING UNIT

# FIG.11

```
        ┌─────────────┐
        │    START    │
        └──────┬──────┘
               ↓
┌────────────────────────────────────────────┐
│   INTRODUCTION OF AMINO ACID  MUTATION      │── SA-1
└──────────────────────┬─────────────────────┘
                       ↓
┌────────────────────────────────────────────┐
│  CALCULATION OF SOLVATION ENTROPY CHANGE AT │── SA-2
│ STRUCTURAL OPTIMIZATION TRANSMEMBRANE SEGMENT│
└──────────────────────┬─────────────────────┘
                       ↓
┌────────────────────────────────────────────┐
│  CALCULATION OF DIFFERENCE IN ENTROPY CHANGE│── SA-3
│       BETWEEN WILD TYPE AND MUTANT          │
└──────────────────────┬─────────────────────┘
                       ↓
┌────────────────────────────────────────────┐
│ EXTRACTION OF THERMOSTABILIZED MUTANT CANDIDATE │── SA-4
└──────────────────────┬─────────────────────┘
                       ↓
               ┌─────────────┐
               │     END     │
               └─────────────┘
```

# FIG.12

WILD TYPE

+····+     $-\Delta S_w$

SEPARATED STATE        PACKED STATE

MUTANT

+···+     $-\Delta S_m$

# FIG.13

+····+

FIG.14

FIG.15

X-RAY CRYSTAL
STRUCTURE

STRUCTURE AFTER
HYDROGENATION

HYDROGENATION

STRUCTURE <1>

# FIG.16

# FIG.17

# FIG.18

STRUCTURE <1>

S3-1 STRUCTURAL OPTIMIZATION

S3-2 EXTRACTION OF TRANSMEMBRANE SEGMENT

$-S_w$

S3-5 $-\Delta S_w = -S_w - (-S'_w)$

S3-5

$-S'_w$

S3-3 EXTRACT TRANSMEMBRANE REGION ALONE AND SEPARATE HELIX

$+\cdots+$

S3-4 STRUCTURAL OPTIMIZATION

$+\cdots+$

S3-a AMINO ACID SUBSTITUTION

STRUCTURE <4>

S3-b STRUCTURAL OPTIMIZATION

S3-c EXTRACTION OF TRANSMEMBRANE SEGMENT

$-S_m$

S3-f

$-\Delta S_m = -S_m - (-S'_m)$

S3-f

$-S'_m$

S3-d EXTRACT TRANSMEMBRANE REGION ALONE AND SEPARATE HELIX

$+\cdots+$

S3-e STRUCTURAL OPTIMIZATION

$+\cdots+$

# FIG.19

STRUCTURE <1>

S4-1 STRUCTURAL OPTIMIZATION

S4-2 EXTRACTION OF TRANSMEMBRANE SEGMENT

$-S_w$

S4-5

$-\Delta S_w = -S_w - (-S'_w)$

S4-5

$-S'_w$

S4-3 EXTRACT TRANSMEMBRANE SEGMENT ALONE AND CREATE EXTENDED STRUCTURE

$+\cdots+$

S4-4 STRUCTURAL OPTIMIZATION

$+\cdots+$

S4-a AMINO ACID SUBSTITUTION

STRUCTURE <4>

S4-b STRUCTURAL OPTIMIZATION

S4-c EXTRACTION OF TRANSMEMBRANE SEGMENT

$-S_m$

S4-f

$-\Delta S_m = -S_m - (-S'_m)$

S4-f

$-S'_m$

S4-d EXTRACT TRANSMEMBRANE SEGMENT ALONE AND CREATE EXTENDED STRUCTURE

$+\cdots+$

S4-e STRUCTURAL OPTIMIZATION

$+\cdots+$

# FIG.20

EP 3 163 481 B1

# FIG.21

# FIG.22

# FIG.23

# FIG.24

# FIG.25

# FIG.26

|  | PROCEDURE 1 | PROCEDURE 2 | PROCEDURE 3 | PROCEDURE 4 | PROCEDURE 5 |
|---|---|---|---|---|---|
| T88E(-) | (-)O | (-)O | (+)× | (+)× | (-)O |
| S91R(-) | (-)O | (-)O | (-)O | (-)O | (-)O |
| C245W(+) | (-)× | (+)O | (+)O | (+)O | (+)O |
| A51W(+) | (-)× | (-)× | (+)O | (+)O | (+)O |
| V239R(+) | (-)× | (-)× | (-)× | (-)× | (-)× |
| NUMBER OF SUCCESSES | 2/5 | 3/5 | 3/5 | 3/5 | 4/5 |

# FIG.27

PROCEDURE 1

# FIG.28

PROCEDURE 2

# FIG.29

## PROCEDURE 3

# FIG.30

## PROCEDURE 4

# FIG.31

PROCEDURE 5

# FIG.32

| | PROCEDURE 1 | PROCEDURE 2 | PROCEDURE 3 | PROCEDURE 4 | PROCEDURE 5 |
|---|---|---|---|---|---|
| A054L | (+)× | (+)× | (+)× | (+)× | (-)O |
| V057A | (-)O | (+)× | (+)× | (-)O | (+)× |
| H075A | (+)× | | (+)× | (+)× | |
| T088A | (-)O | (-)O | (-)O | (-)O | (-)O |
| G114A | | | | | |
| G118A | | | | | |
| T119A | (+)× | | (+)× | (+)× | |
| K122A | (-)O | | (+)× | (-)O | |
| G123A | | | | | |
| P149A | | | | | |
| E151A | | | | | |
| G152A | | | | | |
| A203L | (-)O | | (-)O | (-)O | |
| A204L | (+)× | | (+)× | (+)× | |
| A231L | (+)× | | (+)× | (+)× | |
| L235A | (-)O | | (-)O | (-)O | |
| V239A | (+)× | (+)× | (+)× | (+)× | (+)× |
| NUMBER OF SUCCESSES | 5/11 | 1/4 | 3/11 | 5/11 | 2/4 |

# FIG.33

START

INTRODUCTION OF AMINO ACID MUTATION — SB-1

CALCULATION OF CHANGE OF Λ AT STRUCTURAL OPTIMIZATION MEMBRANE TRANSMEMBRANE SEGMENT — SB-2

CALCULATION OF DIFFERENCE IN CHANGE OF Λ BETWEEN WILD TYPE AND MUTANT — SB-3

EXTRACTION OF THERMOSTABILIZED MUTANT CANDIDATE AFTER COMBINATION WITH ENTROPY COMPONENT — SB-4

END

# FIG.34

ENERGY DECLINE D ASSOCIATED WITH HYDROGEN BOND FORMATION

CENTER-TO-CENTER DISTANCE (Å) BETWEEN ATOMS OF DONOR AND ACCEPTOR

# FIG.35

# FIG.36

|  | PROCEDURE 1 | PROCEDURE 2 | PROCEDURE 3 | PROCEDURE 4 | PROCEDURE 5 |
|---|---|---|---|---|---|
| S91R(-) | (-)O | (-)O | (-)O | (-)O | (-)O |
| S91K(-) | (-)O | (-)O | (-)O | (-)O | (-)O |
| L85R(+) | (-)× | (-)× | (-)× | (-)× | (-)× |
| N280R(-) | (-)O | (-)O | (-)O | (-)O | (-)O |
| N181K(+) | (-)× | (-)× | (-)× | (-)× | (-)× |
| NUMBER OF SUCCESSES | 3/5 | 3/5 | 3/5 | 3/5 | 3/5 |

O:PREDICTION SUCCESS
×:PREDICTION FAILURE

EXPERIMENTAL RESULT, PREDICTION RESULT
(-):STABILIZATION
(+):DESTABILIZATION

# FIG.37

# FIG.38

# FIG.39

# FIG.40

# FIG.41

# FIG.42

|  | PROCEDURE 1 | PROCEDURE 2 | PROCEDURE 3 | PROCEDURE 4 | PROCEDURE 5 |
|---|---|---|---|---|---|
| S91R(-) | (-)O | (-)O | (-)O | (-)O | (-)O |
| S91K(-) | (-)O | (-)O | (-)O | (-)O | (-)O |
| L85R(+) | (-)× | (-)× | (-)× | (-)× | (-)× |
| N280R(-) | (-)O | (-)O | (-)O | (-)O | (-)O |
| N181K(+) | (-)× | (-)× | (-)× | (-)× | (-)× |
| NUMBER OF SUCCESSES | 3/5 | 3/5 | 3/5 | 3/5 | 3/5 |

O:PREDICTION SUCCESS
×:PREDICTION FAILURE

EXPERIMENTAL RESULT,
PREDICTION RESULT
(-):STABILIZATION
(+):DESTABILIZATION

# FIG.43

# FIG.44

# FIG.45

## FIG.46

## FIG.47

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011505800 T

### Non-patent literature cited in the description

- **VADIM CHEREZOV ; DANIEL M. ROSENBAUM ; MICHAEL A. HANSON ; SOREN G. F. RASMUSSEN ; FOON SUN THIAN ; TONG SUN KOBILKA ; HEE-JUNG CHOI ; PETER KUHN ; WILLIAM I. WEIS ; BRIAN K. KOBILKA.** High-Resolution Crystal Structure of an Engineered Human β2-Adrenergic G Protein-Coupled Receptor. *Science,* 2007, vol. 318 (5854), 1258-1265

- Nature of lipid bilayer. LS-EDI Life Science Educational Digital Image Repository. Tokyo University and Center for Structuring Life Science
- *curr. opin. struct. biol.,* 2011, vol. 21, 460-466